# EUROPEAN PATENT SPECIFICATION

(11) **EP 0 451 141 B1**
(45) Date of publication and mention of the grant of the patent: **18.11.1998**
(21) Application number: 89906987.6
(22) Date of filing: 18.05.1989
(51) Int. Cl.: C12Q 1/68

(54) **IMMOBILIZED SEQUENCE-SPECIFIC PROBES**
BEFESTIGUNG VON SEQUENZSPEZIFISCHEN PROBEN
SONDES SPECIFIQUES A DES SEQUENCES IMMOBILISEES

(30) Priority: 20.05.1988 US 197000; 04.05.1989 US 347495
(43) Date of publication of application: 16.10.1991
(73) Proprietor: F. HOFFMANN-LA ROCHE AG, 4002 Basel (CH)
(72) Inventor: SAIKI, Randall, K., Richmond, CA 94805 (US); ERLICH, Henry, A., Oakland, CA 94602 (US)
(86) International application number: US8902170
(87) International publication number: WO8911548

(56) References cited:
- EP-A- 0 130 523
- EP-A- 0 142 299
- Proceedings of the National Academy of Sciences USA, Vol. 81, April 1984, (Washington, D.C., US), G.M. CHURCH et al.: "Genomic Sequencing", pages 1991-1995
- Tetrahedron Letters, Vol. 22, No. 34, 1981, Pergamon Press Ltd (GB), I. SAITO et al.: "Photochemical ring Opening of Thymidine and Thymine in the Presence of Primary Amines", pages 3265-3268
- Nucleic Acids Research, Vol. 14, No. 24, 1986, IRL Press Ltd (Oxford, GB), H.B. GAMPER et al.: "Reverse Southern Hybridization", pages 9943-9954

## Description

This invention relates to nucleic acid chemistry and to methods for detecting particular nucleic acid sequences. More specifically, the invention relates to an assay reagent for diagnostic tests, a method for preparing the assay reagent, a method for detecting the presence of a nucleic acid sequence in a sample with the help of the assay reagent and a kit comprising the assay reagent. The invention has applications in the fields of medical diagnostics, medical microbiology, forensic science, environmental monitoring of microorganisms, food and drug quality assurance, and molecular biology.

Investigational microbiological techniques have been applied to diagnostic assays. For example, Wilson et al., U.S. Patent No. 4,395,486 discloses a method for detecting sickle cell anemia by restriction fragment length polymorphism (RFLP). Wilson et al. identified a restriction enzyme capable of cleaving a normal globin gene but incapable of cleaving the mutated (sickle cell) gene. As sickle cell anemia arises from a point mutation, the method is effective but requires 10 to 20 ml of blood or amniotic fluid.

Various infectious diseases can be diagnosed by the presence in clinical samples of specific DNA sequences characteristic of the causative microorganism or infectious agent. Pathogenic agents include certain bacteria, such as Salmonella, Chlamydia, and Neisseria; viruses, such as the hepatitis, HTLV, and HIV viruses; and protozoans, such as Plasmodium, responsible for malaria. U.S. Patent No. 4,358,535 issued to Falkow et al. describes the use of specific DNA hybridization probes for the diagnosis of infectious diseases. The Falkow et al. method for detecting pathogens involves spotting a sample (e.g., blood, cells, saliva, etc.) on a filter (e.g., nitrocellulose), lysing the cells, and fixing the DNA through chemical denaturation and heating. Then, labeled DNA probes are added and allowed to hybridize with the fixed sample DNA, and hybridization indicates the presence of the pathogen DNA. A problem inherent in the Falkow et al. procedure is insensitivity; the procedure does not work well when very few pathogenic organisms are present in a clinical sample from an infected patient or when the DNA to be detected constitutes only a very small fraction of the total DNA in the sample. Falkow et al. do teach that the sample DNA may be amplified by culturing the cells or organisms in place on the filter.

Routine clinical use of DNA probes for the diagnosis of infectious diseases would be simplified considerably if non-radioactively labeled probes could be employed as described in EP 63,879 to Ward. In the Ward procedure, horseradish peroxidase (HRP) labeled DNA probes are detected by a chromogenic reaction similar to ELISA. The Ward detection methods and reagents are convenient but relatively insensitive, again because the specific sequence that must be detected is usually present in extremely small quantities.

A significant improvement in DNA amplification, the polymerase chain reaction (PCR) technique, was disclosed by Mullis in U.S. Patent No. 4,683,202, and detection methods utilizing PCR are disclosed by Mullis et al. in U.S. Patent No. 4,683,195. In the PCR technique, short oligonucleotide primers are prepared which match opposite ends of a sequence to be amplified. The sequence between the primers need not be known. A sample of DNA or RNA is extracted and denatured, preferably by heat. Then, oligonucleotide primers are added in molar excess, along with dNTPs and a polymerase, preferably Taq polymerase, which is stable to heat and cbmmercially available from Perkin-Elmer/Cetus Instruments. DNA polymerase is "primer-directed," in that replication initiates at the two primer annealing sites. The DNA is replicated, and then again denatured.

This replication results in two "long products," which begin with the respective primers, and the two original strands (per duplex DNA molecule). The products are called "long products," only because there is no defined point of termination of the synthesized strand. The reaction mixture is then returned to polymerizing conditions (e.g., by lowering the temperature, inactivating a denaturing agent, and, if necessary, adding more polymerase), and a second cycle initiated. The second cycle provides the two original strands, the two long products from cycle one, two new long products (replicated from the original strands), and two "short products" replicated from the long products produced in cycle one. The products are called "short products," because these strands must terminate at the 5' end of the "long product" template -- the end defined by the primer that initiated synthesis of the long product. The short products contain the sequence of the target sequence (sense or antisense) with a primer at one end and a sequence complementary to a primer at the other end. On each additional cycle, an additional two long products are produced, and a number of short products, equal to the number of long and short products remaining at the end of the previous cycle, are also produced. Thus, the number of short products can double with each cycle. This exponential amplification of a specific target sequence allows the detection of extremely small quantities of DNA.

The PCR process has revolutionized and revitalized the nucleic acid based medical diagnostics industry. Because the present invention provides reagents that will often be utilized in conjunction with PCR, some additional background information on PCR may be helpful. The PCR process can be used to amplify any nucleic acid, including single or double-stranded DNA or RNA (such as messenger RNA), nucleic acids produced from a previous amplification reaction, DNA-RNA hybrids, or a mixture of any of these nucleic acids. If the original or target nucleic acid containing the sequence variation to be amplified is single stranded, its complement is synthesized by adding one or more primers, nucleotides, and a polymerase; for RNA, this polymerase is reverse transcriptase.

The PCR process is useful not only for producing large amounts of one specific nucleic acid sequence, but also for amplifying simultaneously more than one different specific nucleic acid sequence located on the same or different nucleic acid molecules. When one desires to produce more than one specific nucleic acid sequence in PCR, the appropriate number of different oligonucleotide primers are utilized. For example, if two different specific nucleic acid sequences are to be produced, four primers can be utilized: two for each specific nucleic acid sequence to be amplified.

The specific nucleic acid sequence amplified by PCR can be only a fraction of a larger molecule or can be present initially as a discrete molecule, so that the specific sequence amplified constitutes the entire nucleic acid. In addition, the sequence amplified by PCR can be present initially in an impure form or can be a minor fraction of a complex mixture, such as a portion of nucleic acid sequence due to a particular microorganism that constitutes only a very minor fraction of a particular biological sample. The nucleic acid or acids to be amplified may be obtained from plasmids such as pBR322, from cloned DNA or RNA, or from natural DNA or RNA from sources such as bacteria, yeast, viruses, and higher organisms such as plants or animals. DNA or RNA may be extracted from blood or tissue material such as chorionic villi or amniotic cells by a variety of techniques, including the well known technique of proteolysis and phenol extraction, as is common for preparation of nucleic acid for restriction enzyme digestion. In addition, suitable nucleic acid preparation techniques are described in Maniatis et al., Molecular Cloning: A Laboratory Manual (New York, Cold Spring Harbor Laboratory, 1982), pp. 280-281; U.S. Patent Nos. 4,683,195 and 4,683,202; EP 258,017; and Saiki et al., 1985, Biotechnology 3:1008-1012.

Any specific nucleic acid sequence can be produced by the PCR process. It is only necessary that a sufficient number of bases at both ends of the sequence be known in sufficient detail so that two oligonucleotide primers can be prepared which will hybridize to different strands of the desired sequence at relative positions along the sequence such that an extension product synthesized from one primer, when it is separated from its template (complement), can serve as a template for extension of the other primer. The greater the knowledge about the bases at both ends of the sequence, the greater can be the specificity of the primers for the target nucleic acid sequence, and thus the greater the probability that the process will specifically amplify the target.

The specific amplified nucleic acid sequence produced by PCR is produced from a nucleic acid containing that sequence and called a template or "target." If the target nucleic acid contains two strands, the strands are separated before they are used as templates, either in a separate step or simultaneously with the synthesis of the primer extension products. This strand separation can be accomplished by any suitable denaturing method, including physical, chemical, or enzymatic means. One physical method of separating the nucleic acid strands involves heating the nucleic acid until it is completely (>99%) denatured. Typical heat denaturation involves temperatures ranging from about 80 to 105°C for times ranging from about 1 second to 10 minutes. Strand separation may also be induced by a helicase enzyme, or an enzyme capable of exhibiting helicase activity, e.g., the enzyme RecA, which has helicase activity and in the presence of riboATP is known to denature DNA. The reaction conditions suitable for separating the strands of nucleic acids with helicases are described in Cold Spring Harbor Symposia on Quantitative Biology, Vol. XLIII "DNA: Replication and Recombination" (New York, Cold Spring Harbor Laboratory, 1978), B. Kuhn et al., "DNA Helicases", pp. 63-67, and techniques for using RecA are reviewed in Radding, 1982, Ann. Rev. Genetics 16:405-437.

When the complementary strands of the nucleic acid or acids are separated, whether the nucleic acid was originally double or single stranded, the strands are ready to be used as a template for the synthesis of additional nucleic acid strands. The amplification reaction is generally conducted in a buffered aqueous solution, preferably at a pH of 7 to 9 (all pH values herein are at room temperature) most preferably about pH 8. Preferably, a molar excess (for cloned nucleic acid, usually about 1000:1 primer:template, and for genomic nucleic acid, usually about 10⁶⁻⁸:1 primer:template) of the two oligonucleotide primers is added to the buffer containing the separated template strands. The amount of complementary strand may not be known, however, in many applications, so that the amount of primer relative to the amount of complementary strand may not be determinable with certainty.

The deoxyribonucleoside triphosphates dATP, dCTP, dGTP, and dTTP are also added to the PCR mixture in adequate amounts, and the resulting solution is heated to about 90-100°C for about 1 to 10 minutes, preferably from 1 to 4 minutes. If the target nucleic acid forms secondary structure, the nucleotide 7-deaza-2'-deoxyguanosine-5'-triphosphate is also employed, as is known in the art, to avoid the potential problems such secondary structure can cause. After heating, the solution is allowed to cool to room temperature, preferred for primer hybridization. To the cooled mixture is added a polymerization agent, and the polymerization reaction is conducted under conditions known in the art. This synthesis reaction may occur at temperatures primarily defined by the polymerization agent. Thus, for example, if an E. coli DNA polymerase is used as a polymerizing agent, the maximum temperature for polymerization is generally no greater than about 40°C. Most conveniently, the reaction using E. coli polymerase occurs at room temperature. For most PCR applications, however, the thermostable enzyme Taq polymerase is employed at much higher temperatures, typically 50 to 70°C.

Nevertheless, the polymerization agent for PCR may be any compound or system, including enzymes, which will function to accomplish the synthesis of primer extension products from nucleotide triphosphates. Suitable enzymes for this purpose include, for example, E. coli DNA polymerase I, Klenow fragment of E. coli DNA polymerase I, T4 DNA polymerase, other available DNA polymerases, reverse transcriptase (used in the first cycle of PCR if the target is RNA), and other enzymes, including heat-stable enzymes such as Taq polymerase, which will facilitate combination of the nucleotides in the proper manner to form the primer extension products which are complementary to a target nucleic acid strand. Generally, synthesis will be initiated at the 3' end of each primer and proceed in the 5' direction along the template strand, until synthesis terminates. There may be agents, however, which initiate synthesis at the 5' end and proceed in the other direction, and there seems no reason such agents could not also be used as polymerization agents in PCR.

The newly synthesized strand in PCR is base paired to a complementary nucleic acid strand to form a double-stranded molecule, which in turn is used in the succeeding steps of the PCR process. In the next step, the strands of the double-stranded molecule are separated to provide single-stranded molecules on which new nucleic acid is synthesized. Additional polymerization agent, nucleotides, and primers may be added if necessary for the reaction to proceed. The PCR steps of strand separation and extension product synthesis can be repeated as often as needed to produce the desired quantity of the specific nucleic acid sequence.

As noted above, PCR has revolutionized the nucleic acid based diagnostics industry. European Patent Office Publication 237,362, incorporated herein by reference, discloses assay methods employing PCR. In EP 237,362, PCR-amplified DNA is fixed to a filter and then treated with a prehybridization solution containing SDS, Ficoll, serum albumin, and various salts. A specific oligonucleotide probe (of e.g., 16 to 19 nucleotides) is then added and allowed to hybridize. Preferably, the probe is labeled to allow for detection of hybridized probes. EP 237,362 also describes a "reverse" dot blot, in which the probe, instead of the amplified DNA, is fixed to the membrane.

The recent advent of PCR technology has enabled the detection of specific DNA sequences initially present in only minute (<1 ng) quantities. For example, Higuchi et al., 1988, Nature 332:543-546, describe the characterization of genetic variation between individuals based on samples containing only a single hair. DNA was isolated from the hair by digestion and extraction and then treated under PCR conditions to obtain amplification. Specific nucleotide variations were then detected by either fragment length polymorphism (PCR-FLP), hybridization to sequence-specific oligonucleotide (SSO) probes (a technique also described in Saiki et al., 1986, Nature 324:163-166) or by direct sequencing via the dideoxy method (using amplified DNA rather than cloned DNA). Because PCR results in the replication of a DNA sequence positioned between two primers, insertions and deletions between the primer sequences result in product sequences of different lengths, which can be detected by sizing the product in PCR-FLP. In SSO hybridization, the amplified DNA can be fixed to a nylon filter by UV irradiation in a series of "dot blots" and, in one variation of the technique, then allowed to hybridize with an oligonucleotide probe labeled with HRP under stringent conditions. After excess probe is removed by washing, 3, 3', 5, 5'-tetramethylbenzidine (TMB) and H₂O₂ are added: HRP catalyzes H₂O₂ oxidation of TMB to a blue precipitate, the presence of which indicates hybridized probe. U.S. Patent No. 4,789,630 describes protocols and TMB compounds useful for purposes of the present invention. One may alternatively use one of the other leuco dyes (such as a red leuco dye developed by DuPont and licensed to Kodak) to indicate the presence of HRP. However, any chromogen that develops precipitable color or fluorescence as a consequence of peroxidatic activity can be used to detect HRP-labeled reagents. In fact, any enzyme can be used to label, so long as there exists a colorless substrate which forms a colored or fluorescent product as a result of enzyme activity and the product can be captured on a solid support. Separate dot blot hybridizations are performed for each allele tested.

Church et al., 1984, Proc. Natl. Acad. Sci. USA 81:1991-1995, discloses a method for genomic sequencing which comprises cross-linking restriction enzyme-digested genomic DNA fragments to nylon membranes using UV irradiation, and probing the bound fragments with comparatively long (100-200 bp) DNA probes. Church et al. also discloses that NTPs dried onto nylon membranes and UV irradiated at 0.16 KJ/m2 for two minutes are bound more stably (i.e., TTP = 130x, dGTP = 30x, dCTP = 20x, and dATP = 10x) than non-UV irradiated nucleotides. Primary amino groups are highly reactive with 254 nm light-activated thymine (see Saito et al., 1981, Tetrahedron Lett. 22:3265-68), and this reactivity is believed to be the mechanism by which nucleotides become covalently bound to a membrane.

The detection of genetic variations using SSO probes is typically performed by first denaturing and immobilizing the sample DNA on a nylon or nitrocellulose membrane. The membrane is then treated with short (15-20 base) oligonucleotides under stringent hybridization conditions, allowing annealing only in cases of exact complementarity. A large number of hybridizations must be performed when a sample is examined for the presence of many different sequences. For example, a test for the most common genetic mutations that lead to beta-thalassemia in Mediterranean populations would involve 12 probes and require 12 separate hybridizations, accomplished either by probing one filter 12 times or by conducting simultaneous hybridizations on 12 replicate filters (or some combination thereof). A DNA-based HLA typing test can require 20 to 50 probes and hybridizations, a prohibitive effort if one uses the prior art methods that require either splitting the sample into as many portions as there are probes or blotting the sample followed by probing with a single probe and then removing the probe, a process that must be repeated for each probe tested.

In traditional nucleic acid detection by oligomer hybridization, the DNA in the test sample, including the hybridization target, is noncovalently chemisorbed onto a solid support such as nitrocellulose or nylon and then hybridized to a labeled target-specific probe which, except in the SSO methodology just described, usually contains hundreds to thousands of nucleotides and is made biosynthetically. This method suffers from multiple deficiencies. The noncovalent target capture generally is weak enough that considerable target may be washed from the solid support during detection (see, for example, Gingeras et al., 1987, Nucleic Acids Research 15:5373-5390 and Gamper et al., 1986, Nucleic Acids Research 14:9943-9954). Target chemisorption reduces the reactivity of the target sequence toward hybridization with probe. The capture and hybridization processes normally take many hours to reach completion. The need to chemisorb the target immediately before detection prevents the manufacture of a storage-stable capture reagent with built-in target specificity that can be applied rapidly to test samples. The sequence non-specificity of capture complicates the examination of a single test sample with more than one probe: either a lot of test sample must be available to load different solid supports to incubate with the various probes or a lot of time must be consumed in serial probing of a singly immobilized test sample.

Ranki et al., 1983, Gene 21:77-85, improve on the traditional technology by creating a sequence-specific capture reagent, capturing the target sequence from the test sample by nucleic acid hybridization and increasing specificity by detecting captured target with a second, labeled, sequence-specific nucleic acid probe. However, their technology still suffers from multiple deficits. The sequence-specific capture probe is immobilized by chemisorption, so that the assay still is vulnerable to signal attenuation by desorption of both capture probe and probe-target complex during the incubations and washes. Chemisorption reduces probe reactivity, requiring long incubation times to maximize capture efficiency. Two nucleic acid probes must be manufactured instead of one. The capture and detection probes of Ranki et al. are so large that they must be prepared by biosynthetic instead of much cheaper chemical synthetic routes. A small capture probe would not be immobilized efficiently by chemisorption.

Gingeras et al., 1987, supra, improve further on DNA probe technology by covalently attaching relatively short, chemically synthesized, oligonucleotide hybridization probes to a solid support, dramatically reducing hybridization time. However, direct coupling of the target-specific sequence to the support risks reduced reactivity caused by steric occlusion by the support. Furthermore, the method demonstrated no way of detecting captured DNA apart from the incorporation of radioactive label into the target, a procedure which is relatively hazardous and inconvenient Finally, the beaded solid support of Gingeras et al. is hard to adapt to assays in which multiple targets are probed, because the test sample must be exposed to separate containers of beads carrying the different probes, taking care not to mix beads with different specifications.

Gamper et al., supra, describe a different strategy to accelerate oligomer hybridization: oligo hybridization is performed in solution rather than on a solid support, the hybrid species being simultaneously photochemically trapped, because the target-specific oligomer has been chemically modified with a moiety which crosslinks double-stranded DNA when irradiated. However, apart from the expense of creating the photo-adduct labeling reagent, this method suffers the inconvenience and delay associated with ultrafiltration to remove the considerable excess of unreacted probe, followed by gel electrophoresis to purify the hybridization product to the point that it can be identified. This procedure would be particulary inconvenient to adapt to simultaneous probing of multiple targets, because of the need to engineer targets to be electrophoretically resolvable.

EP 130,523 discloses a solid support which is capable of binding a nucleic acid probe thereto upon suitable irradiation comprising (a) a solid support, (b) a photochemically reaction nuclear acid binding ligand, (c) a divalent radical, chemically linking the substance and the nucleic acid binding ligand and optionally (d) a mutual coupler advantageously making the connection between the solid substrate and the binding ligand. The configuration (a) to (c) or (d) is necessary for performing a solid support with nuclear acid binding sites for the attachment of a nucleic probe by a photochemical reaction. For example, a substrate having hydroxyl groups (cellulose or Sephadex ®) will be transformed to a substrate with photoreactive nuclear acid binding sites, i.e. primary or secondary amino groups. Hence, EP 130,523 is directed to an optional attachment of a nucleic acid probe but not to its hybridization efficiency.

The present invention is directed to enhancing and accelerating the hybridization efficiency of the probe(s).The present invention provides an assay reagent for diagnostic tests, a method for preparing the assay reagent, a method for detecting the presence of a nucleic acid sequence in a sample with the help of the assay reagent and a kit comprising the assay reagent.

The assay reagent for diagnostic tests comprising at least one oligonucleotide probe, optionally an oligodeoxyribonucleotide probe, immobilized on a solid support having reactive groups, said probe comprising a hybridizing region composed of a nucleotide sequence of about 10 to 50 nucleotides complementary to a specific nucleotide sequence to be detected and a spacer arm is characterized in that the immobilization of each hybridizing region is performed via the spacer arm which is longer than said nucleotide sequence and allows the hybridizing region to move away from the solid support and which spacer arm is not able to hybridize to the specific nucleotide sequence to be detected, wherein the spacer arm is covalently bonded at one side to said support and at the other side to the hybridization region.

The spacer arm may be a polynucleotide tail, optionally containing from 200 to 800 nucleotides and, if desired, comprising at least 150 pyrimidine nucleotides, said reactive groups are primary or secondary amine groups and said bonding between said tail and said support being formed by ultraviolet light irradiation. The support may by a nylon support. The hybridizing region is preferably composed of a sequence of nucleotides from 17 to 23 nucleotides in length.

The assay reagent is further characterized in that it comprises a set of probes immobilized on the solid support, wherein said set of probes comprises two or more members, each member of said set having a hybridizing region different from every other member of said set and each member is immobilized on said solid support at a discrete location separate from every other member of said set and, if desired, one member of said set serving as a positive control. In addition, the assay reagent may also comprise a labeled polynucleotide hybridized to one of said probes, wherein said polynucleotide includes at least 50 nucleotides, or comprise a target sequence from a sample hybridized to a probe of said set and a colored or fluorescent compound immobilized on said support at the location of said hybridized probe. The probes of said set may be complementary to nucleic acid sequences of microorganisms or may be complementary to variant alleles of a genetic locus, optionally an HLA locus, especially DQualpha locus.

The method of preparing the assay reagent as defined above is characterized in that a nucleotide sequence of about 10 to 50 nucleotides complementary to a specific nucleotide sequence to be detected is attached by covalent bond to one end of a spacer arm which spacer arm is longer than said nucleotide sequence and is not able to hybridize to the specific nucleotide sequence to be detected, and the other end of the spacer arm is immobilized on a solid support having reactive groups by a covalent bond between the reactive groups and that end of said spacer arm and, if desired, said method comprising (a) contacting said solid support comprising amine groups as reactive groups and (b) irradiating the support prepared in step (a) with ultraviolet light, preferably having a wave-length of 254 nm.

The method for detecting the presence of a nucleic acid sequence in a sample is characterized in that the method comprises: (a) contacting said sample with an assay reagent as described above under conditions that allow for hybridization of complementary nucleic acid sequences; and (b) determining if hybridization has occurred.

The kit comprises the assay reagent as described above.

The present invention provides a particularly advantageous assay method which permits the simultaneous nonisotopic detection of two or more specific nucleic acid sequences or control conditions in a single test sample, using a single solid support divided into discrete regions to which different oligonucleotide probes have been covalently attached via spacer arms. The method comprises:
(a) attaching the probes to defined regions of the solid support through spacer arms, attached at one end to the probe and at the other end to the support;
(b) reacting the test sample with the probe-bearing solid support under conditions promoting hybridization of the probes to any single-stranded complementary nucleic acid sequences in the test sample;
(c) washing away any nucleic acid not hybridized to probe; and
(d) detecting the probe-captured nucleic acid, preferably nonisotopically.
Because of the permanence of covalent attachment, the preparation of immobilized probes can be separated in time from their use, permitting manufacture of a storage-stable detection reagent, the hybridization capture support, which can be used rapidly to detect target nucleic acid sequences in test samples on demand. Covalent probe attachment and the use of a spacer arm between support and probe greatly accelerate and improve the efficiency of hybridization. The use of a dimensionally stable solid support with discrete regions for different probes greatly improves the economics, simplifies the physical format, and increases the reliability of hybridization and detection, because all target sequences in a simple test sample and all control conditions can be probed simultaneously in a single short incubation and because all probe-target hybrids are exposed to identical incubation, wash, and detection conditions. Nonisotopic detection, whether via colored or fluorescent labels directly attached to the target nucleic acid or via colored, fluorescent, or enzyme labels indirectly attached to the target nucleic acid through a specific binding reaction, is much safer and more convenient than detection of radioactive atoms attached to the target nucleic acid, especially when developing storage-stable detection reagents and assay kits.

The invention also relates to a novel, stable, assay reagent comprising oligonucleotide probes covalently attached to discrete regions of a solid support via spacer arms, which probes have sequences designed to hybridize to different analyte nucleic acids in the test sample or to indicate different (positive or negative) control conditions which test the validity of the assay conditions. This assay reagent will have significant commercial impact, being ideally suited to large-scale, automated, manufacturing processes and having a long shelf life. The reagent will prove especially useful in situations where the number of target sequences exceeds the number of samples tested. In general, the greater the number of target sequences and therefore immobilized probes, the greater the improvement of the invention over what has gone before. With PCR-amplified DNA samples, a simple test can easily be assayed for over fifty specific sequences on a single solid support. The nonisotopic detection aspect of the invention is especially well suited to target sequences generated by PCR, a process which permits covalent attachment to all target molecules of colored or fluorescent dyes and of binding moieties like biotin, of colored or fluorescent and of binding moieties like biotin, digoxin, and specific nucleic acid sequences. The methods and reagents of the invention are also suited to detection of isotopically labeled nucleic acid, although this mode is not preferred.

An important aspect of the invention relates to a specific chemistry for attaching oligonucleotide probes to a solid support in a way which is especially suitable to large-scale manufacture and which permits maximization of probe retention and hybridization efficiency. This chemistry comprises covalent attachment of a polynucleotide (preferably poly-dT) tail to the probe and fixation by the ultraviolet irradiation of the photoreactive tailed probe to a solid support bearing primary or secondary amines (e.g., a nylon membrane). However, the invention provides numerous alternative, non-photochemical ways to attach probe to support, wherein electrophilic reagents are used to couple the probe to the spacer and the spacer to the solid support in either reaction order, and wherein the spacer can be any of a large variety of organic polymers or long-chain compounds.

Another aspect of the invention relates to a DNA sequence detection kit, which kit comprises the stable assay reagent, essentially a solid support having oligonucleotide probes covalently bound thereto via a spacer arm. The kit can also include PCR reagents, including PCR primers selected for amplification of DNA sequences capable of hybridizing with the oligonucleotide probes.

To aid in understanding and describing the invention, the following terms are defined below:

"Allele-specific oligonucleotide" (ASO) refers to a probe that can be used to distinguish a given allelic variant from all other allelic variants of a particular allele by hybridization under sequence-specific hybridization conditions.

"DNA polymorphism" refers to the condition in which two or more different variations of a nucleotide sequence exist in the same interbreeding population.

"Genetic disease" refers to specific deletions and/or mutations in the genomic DNA of an organism that are associated with a disease state and include sickle cell anemia, cystic fibrosis, alpha-thalassemia, beta-thalassemia, and the like.

"Label" refers to any atom or molecule which can be used to provide a detectable (preferably quantifiable) signal and which can be attached to a nucleic acid or protein. Labels may provide signals detectable by fluorescence, radioactivity, colorimetry, X-ray diffraction or absorption, magnetism, enzymatic activity, and the like. Suitable labels include fluorophores, chromophores, radioactive atoms (particularly ³²P and ¹²⁵I), electron-dense reagents, enzymes, and ligands having specific binding partners. Enzymes are typically detected by their activity. For example, HRP can be detected by its ability to convert diaminobenzidine (more preferably, however, TMB is used) to a blue pigment, quantifiable with a spectrophotometer. It should be understood that the above description is not meant to categorize the various labels into distinct classes, as the same label may serve in several different modes. For example, ¹²⁵I may serve as a radioactive label or as an electron-dense reagent. HRP may serve as enzyme or as antigen for an antibody, such as a monoclonal antibody (MAb). Further, one may combine various labels for desired effect For example, MAbs and avidin can be labeled and used in the practice of this invention. One might label a probe with biotin, and detect its presence with avidin labeled with ¹²⁵I or with an antibiotin MAb labeled with HRP. Alternatively, one may employ a labeled MAb to dsDNA (or hybridized RNA) and thus directly detect the presence of hybridization without labeling the nucleic acids.

"Oligonucleotide" refers to primers, probes, oligomer fragments, oligomer controls, and unlabeled blocking oligomers and is a molecule comprised of at least two or more deoxyribonucleotides or ribonucleotides. An oligonucleotide can also contain nucleotide analogues, such as phosphorothioates and alkyl phosphonates, and derivatized (i.e., labeled) nucleotides. The exact size of an oligonucleotide will depend on many factors, which in turn depend on the ultimate function or use of the oligonucleotide.

"Primer" refers to an oligonucleotide, whether occurring naturally or produced synthetically, which is capable of acting as a point of initiation of synthesis when placed under conditions in which synthesis of a primer extension product complementary to a nucleic acid strand can occur. The primer is preferably an oligodeoxyribonucleotide and is single stranded for maximum efficiency in amplification, but may alternatively be double stranded. If double stranded, the primer is first treated to separate its strands before being used to prepare extension products. The primer must be sufficiently long to prime the synthesis of extension products in the presence of the polymerase, but the exact length of a primer will depend on many factors. For example, for diagnostics applications, the oligonucleotide primer typically contains 15 to 25 nucleotides. Short primer molecules generally require cooler temperatures to form sufficiently stable hybrid complexes with template. Suitable primers for amplification are prepared by means known to those of ordinary skill in the art, for example by cloning and restriction of appropriate sequences, direct chemical synthesis, and purchase from a commercial supplier. Chemical methods for primer synthesis include: the phosphotriester method described in Narang et al., 1979, Meth. Enzymol. 68:90 and U.S. Patent No. 4,356,270; the phosphodiester method disclosed in Brown et al., 1979, Meth. Enzymol. 68:109; the diethylphosphoramidite method disclosed in Beaucage et al., 1981, Tetrahedron. Lett. 22:1859-1862; and the solid support method disclosed in U.S. Patent No. 4,458,066. The primers may also be labeled, if desired.

"Restriction fragment length polymorphism" (RFLP) refers to a DNA polymorphism at a restriction enzyme recognition site. The restriction enzyme specific for the polymorphic site can be used to digest sample DNA, and when the digested DNA is fractionated by electrophoresis and, if necessary, treated for visualization, different samples produce different restriction endonuclease patterns, depending on the particular polymorphic sequence present in the sample.

"Sequence-specific hybridization" refers to strict hybridization conditions in which exact complementarity between probe and sample target sequence is required for hybridization to occur. Such conditions are readily discernible by those of ordinary skill in the art and depend upon the length and base composition of the probe. In general, one may vary the temperature, pH, ionic strength, and concentration of chaotropic agent(s) in the hybridization solution to obtain conditions under which substantially no probes will hybridize in the absence of an "exact match." For hybridization of probes to bound DNA, the empirical formula for estimating optimum temperature under standard conditions (0.9 M NaCl) is: T(°C) = 4 (N_{G} + N_{C}) + 2(N_{A} + N_{T}) - 5°C, where N_{G}, N_{C}, N_{T}, and N_{A} are the numbers of G, C, A, and T bases in the probe (J. Meinkoth et al., 1984, Analyt. Biochem. 138:267-284). Those of skill in the art recognize, however, that this calculation only gives an approximate value for optimum temperature, which should then be empirically tested to obtain the true optimum temperature. The probe utilized in a sequence-specific hybridization is called a "sequence-specific oligonucleotide" (SSO), which can also be an allele-specific oligonucleotide (ASO). Those of skill in the art recognize that for a single mismatch between probe and target to be destabilizing, the hybridizing region of the probe must be relatively short, generally no longer than about 23 bases, and usually about 17 to 23 bases in length.

"Specific binding partner" refers to a protein capable of binding a ligand molecule with high specificity, as for example in the case of an antigen and an antibody or MAb specific therefor. Other specific binding partners include biotin and avidin, streptavidin, or an anti-biotin antibody; IgG and protein A; and the numerous receptor-ligand couples known in the art.

To aid in understanding the invention, several Figures accompany the description of the invention. These Figures are briefly described below.

Figure 1 depicts a plot of probe binding as a function of UV exposure.

Figure 2 depicts a plot of hybridization efficiency as a function of UV exposure.

Figure 3 depicts a series of dot blots demonstrating the presence of either normal beta-globin or sickle cell beta-globin, obtained by sandwich assay.

Figure 4 depicts a series of dot blots demonstrating the presence of either normal beta-globin or sickle cell beta-globin, obtained by direct assay.

Figure 5 depicts a series of dot blots demonstrating HLA DQalpha genotyping.

Figure 6 depicts a series of dot blots demonstrating beta-thalassemia typing.

The present invention provides a method for detecting the presence of a specific nucleotide sequence in a sample by contacting the sample with immobilized oligonucleotide probes under conditions that allow for hybridization of complementary nucleic acid sequences. In one embodiment of the method, the test sample is contacted with a solid support upon which are immobilized probes specific for one or more target sequences (the "analyte"), probes for a positive control sequence that should be present in all test samples, and optionally probes for a negative control sequence that should not be present in any test sample -- each different probe is immobilized at a distinct region on the solid support. If an analytical signal is detected in the negative control region or if no analytical signal is detected in the positive control region, then the validity of the response in the analyte region is suspect, and the sample should be retested. In another preferred embodiment, a number of different analyte-specific probes are covalently attached to distinct regions of a solid support so that one can test for allelic variants of a given genetic locus in a single hybridization reaction. In still another preferred embodiment, the various analyte-specific probes are complementary to nucleotide sequences present in various microorganisms.

The immobilized probes are also an important aspect of the invention. The probes comprise two parts: a hybridizing region composed of a nucleotide sequence of about 10 to 50 nucleotides (nt) and a spacer arm, at least as long as the hybridizing region, which is covalently attached to the solid support and which acts as a "spacer", allowing the hybridizing region of the probe to move away from the solid support, thereby improving the hybridization efficiency of the probe. In a preferred embodiment, the spacer arm is a sequence of nucleotides, called the "tail", that serves to anchor the probes to the solid support via covalent bonds between nucleotides within the tail of the probe and reactive groups within the solid support matrix.

As described more fully below, the immobilized probes of the invention avoid the problems inherent in prior art detection methods with immobilized probes. These problems include a lack of sensitivity, for many prior art methods for immobilizing probes actually result in the hybridizing region of the probe becoming attached to the solid support and thus less free to hybridize to complementary sequences in the sample. In addition, the prior art methods for synthesizing and attaching the spacer to the probe and to the solid support are complicated, time-consuming, expensive, and often involve the use of toxic reagents. In marked contrast, a preferred method of the invention for synthesizing and immobilizing probes is quickly completed with readily available, relatively nontoxic, reagents, and with practically no chemical manipulations. The polynucleotide tails of the invention are composed of nucleotides that are attached to the hybridizing region with an enzyme or with the aid of commercially available nucleic acid synthesizers. The tails of the invention are attached to the solid support by a similarly problem-free method: exposure to ultraviolet (UV) light.

Those of skill in the art recognize that nucleic acid hybridization serves as the basis for a number of important techniques in the medical diagnostics and forensics industries. In addition, nucleic acid hybridization serves as an important tool in the laboratories where scientific advances in many diverse fields occur. The present invention represents an important step in making nucleic acid based diagnostics even more powerful and useful. As noted above, PCR has played an important role in these same industries and laboratories, and the present invention will often be practiced on samples in which the nucleic acid has been amplified by PCR. Various useful embodiments of the present invention are described below, but the full scope of the invention can only be realized when understood and utilized by the various and diverse practitioners of nucleic acid based diagnostics.

One very important use of the present invention relates to the detection and characterization of specific nucleic acid sequences associated with infectious diseases, genetic disorders, and cellular disorders, including cancer. In these embodiments of the invention, amplification of the target sequence is again useful, especially when the amount of nucleic acid available for analysis is very small, as, for example, in the prenatal diagnosis of sickle cell anemia using DNA obtained from fetal cells. Amplification is particularly useful if such an analysis is to be done on a small sample using non-radioactive detection techniques which may be inherently insensitive, or where radioactive techniques are being employed but where rapid detection is desirable.

The immobilized probes provided by the present invention not only are useful for detecting infectious diseases and pathological abnormalities but also are useful in detecting DNA polymorphisms not necessarily associated with a pathological state. The term forensic is most often used in a context pertaining to legal argument or debate. Individual identification on the basis of DNA type is playing an ever more important role in the law. For example, DNA typing can be used in the identification of biological fathers and so serves as an important tool for paternity testing. DNA typing can also be used to match biological evidence left at the scene of a crime with biological samples obtained from an individual suspected of committing the crime. In a similar fashion, DNA typing can be used to identify biological remains, whether those remains are a result of a crime or some non-criminal activity. The practice of forensic medicine now routinely involves the use of DNA probes, in protocols that can be made more efficient by employing the present invention.

To achieve the important and diverse benefits of the present invention, one must first synthesize the probes to be immobilized on a solid support. The probe sequence can be synthesized in the same manner as any oligonucleotide, and a variety of suitable synthetic methods were noted above in the discussion of PCR primers. The hybridizing region of the probes of the invention is typically about 10 to 50 nt in length, and more often 17 to 23 nt in length, but the exact length of the hybridizing region will of course depend on the purpose for which the probe is used. Often, for reasons apparent to those of skill in the art, the hybridizing region of the probe will be designed to possess exact complementarity with the target sequence to be detected, but once again, the degree of complementarity between probe and target is somewhat tangential to the present invention. The probes of the invention are, however, preferably "tailed" with an oligonucleotide sequence that plays a critical role in obtaining the benefits provided by the present invention.

This tail of the probes of the invention consists of ribonucleotides or deoxyribonucleotides (e.g., dT, dC, dG, and dA). The nucleotides of the tail can be attached to the hybridizing region of the probe with terminal deoxynucleotidyl transferase (TdT) by standard methods. In addition, the entire tailed probe can be synthesized by chemical methods, most conveniently by using a commercially available nucleic acid synthesizer. One can also synthesize the tails and hybridizing regions separately and then combine the two components. For instance, a preparation of tails can be prepared (and even attached to a solid support, such as a bead) and then attached to a preparation of hybridizing regions.

When using a DNA synthesizer to make the tailed probes of the invention, one should take steps to avoid making a significant percentage of molecules that, due to a premature chain termination event, do not contain a hybridizing region. One such step involves synthesizing the hybridizing region of the probe first, creating a tailed probe with the hybridizing region at the 3' end of the molecule. Because the likelihood of a premature chain termination event increases with the length of the molecule, this step increases the likelihood that if a chain termination event occurs, the occurrence merely results in a shorter tail. However, because most premature chain termination events are a result of a failure to "de-block" during synthesis, and because the exact number of residues in the tail of a probe of the invention is not critical, one may also merely omit the blocking and de-blocking steps during automated synthesis of the tail region of the probe. If these steps are omitted (during tail synthesis only), the tail can be placed on either the 5' or 3' end of the probe with equal efficiency and satisfactory results.

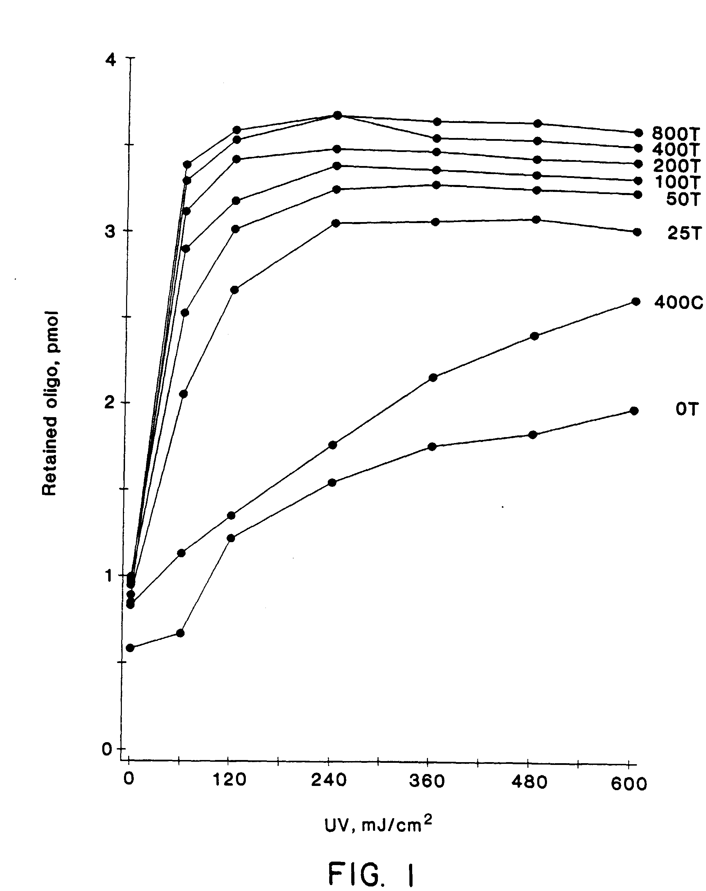

As noted above, the preferred spacer arms of the probes of the invention are comprised of nucleotide tails. Because the tails serve to attach the probe to the solid support, the relative efficiency with which a given oligonucleotide will react with a solid support is important in choosing the sequence to serve as the tail in the probes of the invention. Most often, the tail will be a homopolymer, and Figure 1, below, depicts the relative efficiencies with which synthetic oligonucleotides with varying length homopolymer tails were covalently bound to a nylon filter as a function of UV exposure. As shown in the figure, oligonucleotides with longer poly-dT tails were more readily fixed to the membrane, and all poly-dT tailed oligonucleotides attained their maximum values by 240 mJ/cm² of irradiation at 254 nm. In contrast, the poly-dC (400 nt in length) tail required more irradiation to crosslink to the membrane and was not comparable to the equivalent poly-dT tail even after 600 mJ/cm² exposure. Untailed oligonucleotides were retained by the filter in a manner roughly parallel to that of the poly-dC-tailed probes.

Thus, the probes of the invention preferably comprise a poly-dT tail of greater than 10 thymidine (T) residues. Usually, the tail will comprise at least 100 T residues, and most preferably, the tail will comprise at least 400 dT nucleotides. As the poly-dT tail functions primarily to bind the probe to the solid support, the exact number of dT nucleotides is not critical, as noted above. Although those of skill in the art will readily recognize the fact, it should be noted that the composition of the tail need not be homogeneous, i.e., a mixture of nucleotides may be used. Preferably, however, the tail will include a significant number of thymine bases, as T reacts most readily with the solid support by the preferred methods for making the immobilized probes of the invention, which methods are discussed more fully below. If one desires to utilize the probes in sequence-specific hybridizations, one must be aware of the problem caused by creation of a random sequence in the heterogeneous tail that closely resembles the hybridizing region of a probe. If a heterogeneous tail is employed, it is still desirable to maintain a distribution of 150 pyrimidine residues per tail, if the probes are to be fixed to a solid support by UV irradiation.

The tail should always be larger than the hybridizing region, and the greater the disparity in size between the hybridizing region and the tail (so long as the tail is larger), the more likely it is that the tail, rather than the hybridizing region, will react with the support, a preferred condition. Larger tails thus increase the likelihood that only the tail will participate in reacting with and thereby binding to the solid support. Because the tail also functions as a "spacer," enabling the complementary sequence to diffuse away from the solid support where it may hybridize more easily, free of steric interactions, larger tails are doubly preferred. Excessively extended tails, however, are uneconomical, and, if carried to an extreme, excessive tailing could have adverse effects.

A preferred method of synthesizing a probe of the invention is as follows. The probe is synthesized on a DNA synthesizer (the Model 8700, marketed by Biosearch, is suitable for this purpose) with beta-cyanoethyl N, N-diisopropyl phosphoramidite nucleosides (available from American Bionetics) using the protocols provided by the manufacturer. If desired, however, only the hybridizing region of the probe is synthesized on the instrument, and then 200 pmol of the probe are tailed in 100 µl of reaction buffer at pH = 7.6 and containing 100 mM cacodylate, 25 mM Tris-base, 1 mM CoCl₂, and 0.2 mM dithiothreitol with 5 to 160 nmol deoxyribonucleotide triphosphate (dTTP) and 60 units (50 pmol) of terminal deoxyribonucleotidyl transferase (available from Ratliff Biochemicals) for 60 minutes at 37 degrees C (see Roydhoudhury et al., 1980, Meth. Enz. 65:43-62, for buffer preparation). Reactions are conveniently stopped by the addition of 100 µl of 10 mM EDTA. The lengths of tails can be controlled by limiting the amount of dTTP (or other nucleotide) present in the reaction mixture. For example, a nominal tail length of 400 dT residues is obtained by using 80 nmol of dTTP in the protocol described above.

Once the tailed probe of the invention is synthesized, the probe is then attached to a solid support. Suitable solid supports for purposes of the present invention will contain (or can be treated to contain) free reactive primary or secondary amino groups capable of binding a UV-activated pyrimidine, especially thymine. Secondary amino groups may be preferred for purposes of the present invention. There are many ways to assure that a solid support (not necessarily nylon) has free, particularly secondary, amino groups. Amine-bearing solid supports suitable for purposes of the present invention include polyethylenimine (chemisorbed to any solid, such as cellulose or silica with or without glutaraldehyde crosslinking) and silica or alumina or glass silanized with amine-bearing reagents such as PCR Inc.'s Prosil^{TM} 220, 221, 3128, and 3202 reagents. Manville sells controlled porosity glass papers (Biomat^{TM}) appropriate for aminoalkyl silanization. One may alkylate immobilized primary amines (e.g. with a methyl halide or with formaldehyde plus cyanoborohydride, (as described by Jentoff et al., 1979, J. Biol. Chem. 254:4359-4365). As noted above, one may use a solid support to which polyethylenimine has been chemisorbed. Polyvinyl chloride sheets containing PEI-loaded silica are commercially available (manufactured by Amerace and sold by ICN as Protrans^{TM} and by Polysciences as Poly/Sep^{TM}), and PEI loading of cellulose is well known.

The solid support, also called a substrate, can be provided in a variety of forms, including membranes, rods, tubes, wells, dipsticks, beads, ELISA-format plates, and the like. A preferred support material is nylon, which contains reactive primary amino groups and will react with pyrimidines irradiated with UV light. Preferred solid supports include charge modified nylons, such as the Genetrans-45^{TM} membrane marketed by Plasco and the ZetaProbe^{TM} membrane marketed by Bio-Rad.

Having chosen a suitable solid support, one makes the preferred immobilized probes of the invention by reacting a tailed oligonucleotide probe with the solid support under conditions that favor covalent attachment of the tail to the solid support. In a preferred embodiment, the solid support is a membrane, and probe binding results from exposure of the probe on the membrane to UV irradiation, which activates the nucleotides in the tail, and the activated nucleotides react with free amino groups within the membrane. Careful dessication of tailed probes of the invention spotted onto a suitable solid support can also be used to facilitate covalent attachment of the probes to the substrate. One can assay for the presence or absence in a solid support matrix of reactive groups capable of reacting with oligonucleotides by the procedure described in Example 1.

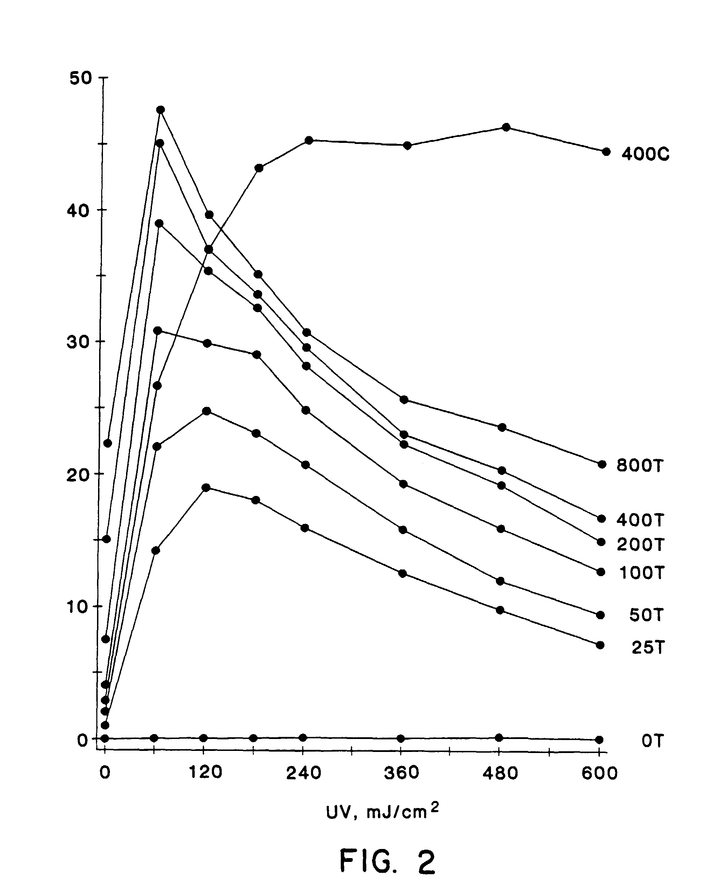

As is apparent from the foregoing, a preferred method for preparing the immobilized probes of the invention comprises fixing an oligonucleotide probe with a poly-dT tail to a nylon membrane by UV irradiation. Although poly dT tails react very efficiently to solid supports by the methods of the present invention, efficiency of reaction of oligonucleotides with a membrane does not necessarily correlate with hybridization efficiency. One may therefore wish to determine the hybridization efficiency of a given oligonucleotide probe after immobilization on a solid support. When the hybridization of various tailed probes is measured as a function of UV dosage, as shown in Figure 2, one observes that the optimum exposure changes with length of a poly-dT tail. Optimal exposures are about 20 mJ/cm² for 800 nt poly-dT tails and about 40 mJ/cm² for 400 nt poly-dT tails.

At 60 mJ/cm² exposure, one observes that oligonucleotides with longer tails hybridize more efficiently than can be accounted for by the additional amounts of probe reacted with and bound to the filter. This increased efficiency is believed to be due to a spacing effect: increasing the distance between the membrane and the hybridizing region of the immobilized probe may increase hybridization efficiency of the probe. Thus, too much UV exposure during immobilization can not only damage the nucleotides in the probe but also can reduce the average spacer length and decrease hybridization efficiency. It is important to note that because dC tails react less efficiently (as compared to dT tails) with a membrane, hybridization efficiency of a poly-dC tailed probe reaches a plateau where loss due to UV damage and tail shortening is compensated for by the fixing of new molecules to the membrane (see Figures 1 and 2). This characteristic of poly-dC tails may make such tails preferred when UV exposure cannot be carefully controlled.

No matter what the base content of the tail of the probe, one may automate the attachment of probe to support in accordance with the method of the present invention. One semi-automated means of attachment preferred for positive charge nylon membranes is as follows. A commercially available "dot-blot" apparatus can be readily modified to fit into a Perkin-Elmer/Cetus Pro/Pette® automated pipetting station; the membrane is then placed on top of the dot-blot apparatus and vacuum applied. The membrane dimples under the vacuum so that a small volume (5 to 20 µl) of probe applied forms a consistent dot with edges defined by the diameter of the dimple. No disassembly of the apparatus is required to place and replace the membrane -- the vacuum may be kept constant while membranes are applied, spotted with probe, and removed.

Once the probes are spotted onto the membrane, the spotted membrane is treated to immobilize the probes. A preferred method for covalently attaching the probes to a nylon membrane is as follows. Tailed oligonucleotides in TE buffer (10 mM Tris-HCl, pH = 8.0, and 0.1 mM EDTA) are applied to a Genetrans-45^{TM} (Plasco) membrane with a BioDot^{TM} (Bio-Rad) spotting manifold. The damp membranes, also called "filters," are then placed on paper pads soaked with TE buffer, the pads and filters are then placed in a UV light box (the Stratalinker 1800^{TM} light box marketed by Stratagene is suitable for this purpose) and irradiated at 254 nm under controlled exposure levels. UV dosage can be controlled by time of exposure to a particular UV light source or, more preferably, by measuring the radiant UV energy with a metering unit. Exposure time typically ranges from about 0.1 to 10 minutes, most often about 2 to 3 minutes. The support is preferably damp during irradiation, but if the support is dried first, a shorter UV irradiation exposure can be used. The irradiated filters are washed in a large volume of a solution composed of 5X SSPE (1X SSPE is 180 mM NaCl, 10 mM NaH₂PO₄, and 1 mM EDTA, pH = 7.2) and 0.5% sodium dodecyl sulfate (SDS) for about one-half hour at 55 degrees C to remove unreacted oligonucleotides. Filters can then be rinsed in water, air dried, and stored at room temperature until needed.

UV irradiation of nucleotides is known to cause pyrimidine photochemical dimerization, which, for purposes of the present invention, is not preferred. A number of steps can be taken to reduce dimerization during UV irradiation, including: applying the oligonucleotide probe to the membrane at a high pH, above 9 and preferably above 10; applying the probe to the membrane at a very low ionic strength, between 0 and 0.01; using the lowest probe concentration that gives the desired signal intensity; and irradiating with light excluding wavelengths longer than 250 nm, preferably with no light with a wavelength longer than 240 nm. However, the extent to which these steps could impair probe immobiliziation has not been tested. In general, spotting and attachment of the probe to the membrane should be done at a temperature and in a solvent that minimizes base-pairing and base-stacking in the probes.

After constructing the novel immobilized probes of the invention, one is ready to employ those probes in the useful nucleic acid sequence detection methods of the invention. In a preferred embodiment of this method, a sample suspected of containing a target nucleic acid sequence is treated under conditions suitable for amplifying the target sequence by PCR. Note that the process of "asymmetric" PCR, described by Gyllensten and Erlich, 1988, Proc. Natl. Acad. Sci. USA 85:7652-7656, for generation of single stranded DNA can also be used to amplify the sample nucleic acid. The PCR primers are biotinylated, for subsequent detection of hybridized primer-containing sequences. The amplification reaction mixture is denatured, unless asymmetric PCR was used to amplify, and then applied to a membrane of the present invention under conditions suitable for hybridization to occur (most often, sequence-specific hybridization). Hybridized probe is detected by binding streptavidin-horseradish peroxidase (SA-HRP, available from a wide variety of chemical vendors) to the biotinylated DNA, followed by a simple colorimetric reaction in which a substrate such as TMB is employed. One can then determine whether a certain sequence is present in the sample merely by looking for the appearance of colored dots on the membrane.

In an especially preferred embodiment of the above method, a filter with immobilized oligonucleotides is placed in hybridization solution containing 5X SSPE, 0.5% SDS, and 100 ng/ml SA-HRP (as marketed under the SeeQuence^{TM} by Cetus and Eastman Kodak). PCR-amplified sample DNA is denatured by heat or by addition of NaOH and EDTA and added immediately to the hybridization solution, which contains enough SSPE to neutralize any NaOH present. The sample is then incubated at a suitable temperature for hybridization to occur (typically, as exemplified below, at 55 degrees C for 30 minutes). During this incubation, hybridization of product to immobilized oligonucleotide occurs as well as binding of SA-HRP to biotinylated product. The filters are briefly rinsed in 2X SSPE and 0.1% SDS at room temperature, then washed in the same solution at 55 degrees C for 10 minutes, then quickly rinsed twice in 2X PBS (1X PBS is 137 mM NaCl, 2.7 mM KCI, 1.5 mM KH₂PO₄, and 8 mM Na₂HPO₄, pH = 7.4) at room temperature. Color development is performed by incubating the filters in red leuco dye or TMB at room temperature for 5 to 10 minutes. Photographs are taken of the filters after color development for permanent records.

Although the detection method described above is preferred, those of skill in the art recognize that the immobilized probes of the invention can be utilized in a variety of detection formats. One such format involves labeling the immobilized probe itself, instead of the sample nucleic acid. If the probe is labeled at or near the end of the hybridizing sequence (far from the site of attachment of the probe to the solid support), one can treat the potentially hybridized sample DNA with an appropriate restriction enzyme, i.e., one that cleaves only duplex nucleic acids at a sequence present in the hybridizing region of the probe, so that restriction releases the label from the probe (and the membrane) for detection of hybridization. Suitable labels include peroxidase enzymes, acid phosphatase, radioactive atoms or molecules (e.g., ³²P, ¹²⁵I, etc.), fluorophores, dyes, biotin, ligands for which specific monclonal antibodies are available, and the like. If the primer or one or more of the dNTPs utilized in a PCR amplification has been labeled (for instance, the biotinylated dUTP derivatives described by Lo et al., 1988, Nuc. Acids Res. 16:8719), instead of the immobilized probe, then, as noted above, hybridization can be detected by assay for presence of label bound to the membrane.

The immobilized probes of the invention can also be used in detection formats in which neither probe nor primer is labeled. In such a format, hybridization can be detected using a labeled "second probe." The second probe is complementary to a sequence occurring within the target DNA, but not overlapping the bound probe sequence; after hybridization of the second probe, the immobilized probe, second probe, and target sequence form a nucleic acid "sandwich," the presence of which is indicated by the presence of the label of the second probe on the membrane. One could also employ monoclonal antibodies (or other DNA binding proteins) capable of binding specifically to duplex nucleic acids (e.g., dsDNA) in a detection format that uses no labeled nucleic acids. Those of skill in the art will recognize that one important advantage of the immobilized probes of the invention is the ability, at least with most detection formats, to recycle the support-bound probe by denaturing the hybridized complex, eluting the sample DNA, and treating the support (for example, by washing, bleaching, etc.) to remove any remaining traces of extraneous DNA, label, developer solution, and immobilized dye (see U.S. Patent No. 4,789,630 and PCT application No. 88/0287.

Those of skill in the art will recognize the many and diverse uses for the immobilized probes of the present invention. One exciting application of these immobilized probes is in conjunction with the technique of simultaneous amplification of several DNA sequences ("multiplex" PCR). Such simultaneous amplification can be used to type at many different loci with a single membrane. For instance, one can type for the polymorphic HindIII site in the ^{G}gamma gene (see Jeffreys, 1979, Cell 18:1-10), the polymorphic AvaII site in the low density lipoprotein receptor gene (see Hobbs et al., 1987, Nuc. Acids Res. 15:379), and for polymorphisms in the HLA DQalpha gene simultaneously by amplifying all three loci in a single PCR and applying the amplified material to a suitable set of immobilized probes of the present invention. Other genetic targets whose analysis would be simplified by this technique include the detection of somatic mutations in the ras genes, where six loci and 66 possible alleles occur (see Verlaan-de Vries et al., 1986, Gene 50:313-320); the typing of DNA polymorphisms at the HLA DP locus; the detection of beta-thalassemia in Middle Eastern populations, where in addition to the endogenous mutations. Mediterranean and Asian Indian mutations are present at significant frequencies; the detection of infectious pathogens; and the detection of microorganisms in environmental surveys.

In many of these applications, it will be desirable to obtain a membrane on which are immobilized a diverse set of oligonucleotides specific for different sequences that nevertheless can hybridize under the same sequence-specific hybridization conditions. If necessary, this situation can be achieved by adjusting the length, position, and strand-specificity of the probes, or by varying the amount of probe applied to the membrane, or by adding a salt, such as tetramethylammonium chloride, to the hybridization buffer to minimize differences among immobilized oligonucleotides caused by varying base compositions (see Wood et al., 1985, Proc. Natl. Acad. Sci. USA 82:1585-1588).

The examples below illustrate various useful embodiments of the invention and enable the skilled artisan to appreciate the invention more fully and so should not be construed as limiting the scope of the invention in any way.

### Example 1

### Probe Retention and Hybridization Efficiency

The stable binding of poly-dT-tailed probe sequences to nylon as a function of tail length and UV exposure was examined as described below. A 19-base oligonucleotide (RS18: 5'-CTCCTGAGGAGAAGTCTGC) was labeled at its 5' end with gamma ³²P-ATP and T4 polynucleotide kinase (see Saiki et al, 1986, Nature 324:163-166). Portions of the kinased probe were then tailed with dTTP and terminal transferase (TdT), as described by Roydhoudhury et al. RS 18 was present at a concentration of 2 µM, TdT (Ratliff Biochemicals, Los Alamos, NM) at 600 U/ml, and either dCTP or dTTP at either 0 µM, 50 µM, 100 µM, 200 µM, 400 µM, or 800 µM to prepare constructs with either dC or dT tails of approximately 0, 25, 50, 100, 200, 400 or 800 dT bases or 400 dC bases per molecule. Reaction mixtures were incubated for 60 minutes at 37°C and were terminated by addition of an equal volume of 10 mM EDTA.

Four pmol of each sample diluted in 100 µl of TE buffer were spotted onto nine duplicate filters (Genetrans-45 nylon, Plasco, Woburn, Mass.), UV irradiated for various times, washed to remove unbound oligonucleotides, and then each spot was measured by scintillation counting to determine the amount of probe crosslinked to the nylon membrane. The values plotted in Figure 1 are relative to an unirradiated, unwashed control filter (100% retention). UV irradiation was accomplished by placing the filters in a Stratalinker 1800^{TM} UV light box and irradiating the filters at 254 mn. Dosage was controlled using the intemal metering unit of the device. The filters were then washed in 5X SSPE, 0.5% SDS for 30 minutes at 55°C to remove DNA not stably bound. The results plotted in Figure 1 show that even the non-tailed probe was retained by the membrane, but that retention of the untailed probe was not greatly improved by UV irradiation. The 400-dT tailed probe exhibited >90% retention after suitable exposure.

However, high retention does not necessarily correlate with high hybridization efficiency. Thus, hybridization efficiency was measured as follows. Probes were prepared with poly-dT tails as above, but with unlabeled RS 18. The probes were spotted onto filters and UV irradiated, and excess probe was washed from the membrane by incubating the membrane in 5X SSPE, 0.5% SDS, for 30 minutes at 55°C. The membranes were then hybridized with 5 pmol of complementary ³²P-kinase labeled 40-mer (RS24: 5'-CCCACAGGGCAGTAACGGCAGACTTCTCCTCAGGAGTCAG, specific activity of 1.5 uCi/pmol) in a solution (10 ml) containing 5X SSPE and 0.5% SDS, at 55°C for 20 minutes, which are sequence-specific hybridization conditions. The membrane was then washed first with 2X SSPE, 0.1% SDS (3 x 100 ml) for 2 minutes at about 25°C, then in 2X SSPE, 0.1% SDS at 55°C for 5 minutes. The individual spots were excised, counted, and the counts plotted against UV exposure, as shown in Figure 2. The values plotted are fmol RS24 hybridized to the membrane. The results show that none of the non-tailed probe was able to hybridize under the conditions used, even though as much as 50% of the applied RS 18 should be bound to the membrane. All of the tailed probes were able to hybridize, with hybridization efficiency increasing with increasing tail length. Optimal UV exposures were from about 60 to 120 mJ/cm².

### Example 2

### Sandwich Assay for Sickle-Cell Anemia

Two allele-specific probes were prepared, one for the normal beta-globin allele, called RS18, and one for the sickle-cell allele, RS21 (5'CTCCTGTGGAGAAGTCTGC); each probe had a 400 nt poly-dT tail. If desired, a probe for the hemoglobin C allele can be prepared with the sequence: 5'CTCCTAAGGAGAAGTCTGC. Eight replicate filters were prepared and spotted with 4, 2, 1, and 0.5 pmol of each tailed probe using the method set forth in Example 1, and then UV irradiated by placing the filters, DNA-side down, directly onto a TM-36 Transilluminator UV light box (U.V. Products, San Gabriel, CA) for 5 minutes. Four 1 µg samples of genomic DNA (from cell lines Molt4 (beta^{A}beta^{A}), SC-1 (beta^{S}beta^{S}), M + S (beta^{A}beta^{S}), and GM2064 (beta^{Δ}beta^{Δ}), a beta-globin deletion mutant) were subjected to 30 PCR amplification cycles with the primer pair PC03 (5'ACACAACTGTGTTCACTAGC) and KM38 (5'TGGTCTCCTTAAACCTGTCTTG).

PCR was carried out in substantial accordance with the procedure described by Saiki et al., 1988, Science 239:1350-1354. The DNA samples were amplified in 100 µl of reaction buffer containing 50 mM KCl, 10 mM Tris-HCl (pH = 8.4), 1.5 mM MgCl₂, 100 µg/ml gelatin, 200 µM each of dATP, dCTP, dGTP, and dTTP, 0.2 µM of each primer, and 2.5 units of Taq DNA polymerase (Perkin-Elmer/Cetus Instruments). The cycling reaction was performed on a programmable heat block, the DNA Thermal Cycler, available from PECI, set to heat at 95 degrees C for 15 seconds (denature), cool at 55 degrees C for 15 seconds (anneal), and incubate at 72 degrees C for 30 seconds (extend) using the Step-Cycle program. After 30 cycles, the samples were incubated an additional 5 minutes at 72 degrees C.

Each amplification product (18 µl) was denatured by heating at 95°C for 5 minutes in 1 ml of TE, and then quenched on ice. A solution (4 ml) of 6.25X SSPE, 6.25X Denhardt's, and 0.625% SDS was mixed with 1 ml of each denatured PCR product, hybridized to one of the filters for 15 minutes at 55°C, washed with 2X SSPE, 0.1% SDS (3 X 100 ml) for 2 minutes at about 25°C, and then washed with 2X SSPE, 0.1% SDS (1 x 100 ml) for 5 minutes at 55°C.

The membranes were then equilibrated in 2X SSPE, 0.1% (v/v) Triton X-100 (100 ml) for 3 minutes at about 25°C to remove SDS. All of the filters were then hybridized in the same buffer with a horseradish peroxidase (HRP) labeled 15-mer, RS 111 (5'-GCAGGTTGGTATCAA), specific for the PC03/KM38 amplification product, prepared by the method disclosed in PCT publications WO 89/02931 and 89/02932, incorporated herein by reference.

These methods essentially involve derivatizing the nucleic acid probe using a linear linking molecule comprising a hydrophilic polymer chain (e.g., polyoxyethylene) having a phosphoramidite moiety at one end and a protected sulfhydryl moiety at the other end. The phosphoramidite moiety couples to the nucleic acid probe by reactions well known in the art (e.g., Beaucage et al., 1981, Tetrahedron Lett. 22: 1859-1862), while the deprotected sulfhydryl group can form disulfide or other covalent bonds with a protein, e.g., HRP. The HRP is conjugated to the linking molecule through an N-maleimido-6-aminocaproyl group. The label is prepared by esterifying N-maleimido-6-aminocaproic acid with sodium 4-hydroxy-3-nitrobenzene sulfonate in the presence of one equivalent of dicylcohexylcarbodiimide in dimethylformamide. After purification, the product is added to phosphate buffer containing HRP at a weight ratio of 8:1 HRP to ester. The oligonucleotide probe is synthesized in a DNA synthesizer, and the linking molecule having the structure (C₆H₅)₃CS-(CH₂CH₂O)₄-P(CH₂CH₂CN) [N(i-Pr)₂] is attached using phosphoramidite synthesis conditions. The trityl group is removed, and the HRP derivative and probe derivative are mixed together and allowed to react to form the labeled probe. A biotin-labeled probe may be prepared by similar methods.

The membrane was incubated with 4 pmol RS 111 in a solution (8 ml) composed of 5X SSPE, 5 x Denhardt's, and 0.5% Triton X-100 for 10 minutes at 40°C. Following incubation, the membrane was washed with 2X SSPE, 0.1% Triton X-100 (3 x 100 ml) for 2 minutes at about 25°C.

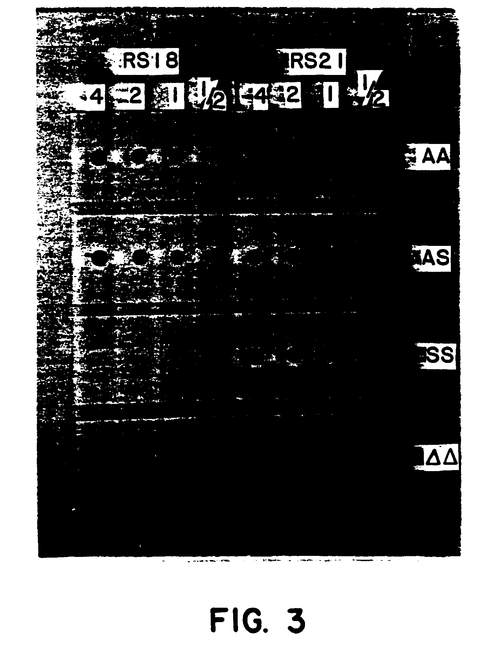

The reaction was followed by color development (Sheldon et al., 1986, Proc. Natl. Acad. Sci. USA 83:9085-9089) with TMB/H₂O₂, as shown in Figure 3. The membranes were soaked in 100 ml of color development buffer B (CDB-B: 237 mM NaCl, 2.7 mM KCI, 1.5 mM KH₂PO₄, 8.0 mM Na₂HPO₄, pH 7.4, 5% (v/v) Triton X-100, 1 M urea, and 1% dextran sulfate), followed by washes with 2 x 100 ml of CDB-C (100 mM sodium citrate, pH 5.0) for 2 minutes at room temperature. Color was developed by replacing the CDB-C solution with 100 ml of CDB-D (100 mM sodium citrate, pH 5.0, 0.1 mg/ml 3,3',5,5'-tetramethylbenzidine), adding 50 µl of 3% H₂O₂, and allowing the color to develop for 30 minutes. The beta-globin genotypes of the amplified DNA samples were readily apparent from the filters, and good signal intensity was obtained even from the 0.5 pmol spot.

### Example 3

### Direct Assay for Sickle-Cell Anemia

A second set of DNAs (as described in Example 2 above) was amplified with PC03 and BW19. BW19 has the sequence 5'CAACTTCATCCACGTTCACC, and is covalently bound to a molecule of biotin at the 5' end. Twelve µl of each of these amplification products were denatured as described in Example 2, added to 4 ml of hybridization buffer (6.25X SSPE, 6.25X Denhardt's, and 0.625% SDS), and incubated with the membrane-bound probe (the remaining four filters from Example 2 above) at 55°C for 15 minutes. The membranes were then washed with 2X SSPE, 0.1% SDS (3 x 100 ml) for 3 minutes at room temperature, followed by a wash with 2X SSPE, 0.1% SDS (1 x 100 ml) for 5 minutes at 55°C.

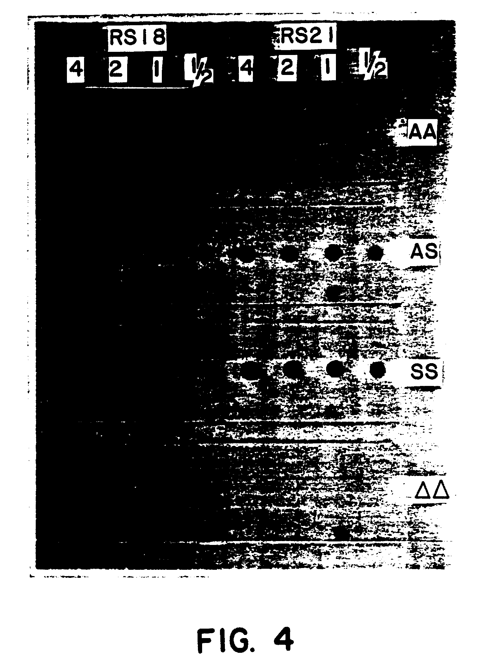

The membranes were pooled together and equilibrated in 100 ml of CDB-A for 5 minutes at about 25°C (CDB-A: 237 mM NaCl, 2.7 mM KCl, 1.5 mM KH₂PO₄, 8.0 mM Na₂HPO₄, pH 7.4, and 5% Triton X-100). The membranes were then placed in a heat-sealable bag with 10 ml of CDB-A and See-Quence^{TM} SA-HRP conjugate (Cetus Corporation, Emeryville, CA) at 0.3 µg/ml and gently shaken for 10 minutes at about 25°C. Excess conjugate was removed by washing with CDB-A (3 x 100 ml for 3 minutes at 25°C), CDB-B (1x 100 ml for 5 minutes at 25°C), and CDB-C (2 x 100 ml for 3 minutes at 25°C). The membranes were then equilibrated in CDB-D (100 ml) for 5 minutes at room temperature, followed by addition of 50 µl of 3% H₂O₂. The color was allowed to develop for 30 minutes with gentle shaking, followed by washing under deionized water for 10 minutes. The four filters after color-development are shown in Figure 4. The intensity and specificity of signals detected by this method compare favorably to those obtained by sandwich hybridization. The faint signal in GM2064 was due to beta-globin contamination in that DNA sample.

### Example 4

### HLA DOalpha Genotyping

The DQalpha test is derived from a PCR-based oligonucleotide typing system that partitions the polymorphic variants at the DQalpha locus into four DNA major types denoted DQA1, DQA2, DQA3, and DQA4, three DQA4 subtypes, DQA4.1, DQA4.2, and DQA4.3, and three DQA1 subtypes, DQA1.1, DQA1.2, and DQA1.3 (see Higuchi et al., 1988, Nature 332:543-546 and Saiki et al., 1986, Nature 324: 163-166).

Four oligonucleotides specific for the major types, four oligonucleotides that characterized the subtypes, and one control oligonucleotide that hybridizes to all allelic DQalpha sequences were given 400 nt poly-dT tails and spotted onto 12 duplicate nylon filters. About 2 to 10 pmol of each probe were placed in each spot

With regard to amount of probe spotted, however, one may wish to employ lower amounts of RH54 and GH64, i.e., 0.035 pmol RH54 per spot is preferred. These probes are positive control probes for amplifcation and will hybridize to any DQalpha alleles under the conditions described. By reducing the amount of positive control probe on the membrane, one can make the positive control probe the least sensitive probe on the membrane. Then, if insufficient amplified DQalpha DNA is applied to the membrane, one can recognize the problem, for the positive control probe will not react or will react only very weakly. Otherwise, when insufficient sample DNA is applied to the membrane, one runs the risk of misreading a heterozygous type as a homozygous type, because some probes hybridize less efficiently than others.

After spotting, the membranes were irradiated at 40 mJ/cm². The sequences of the hybridizing regions of the resulting immobilized probes are shown below.

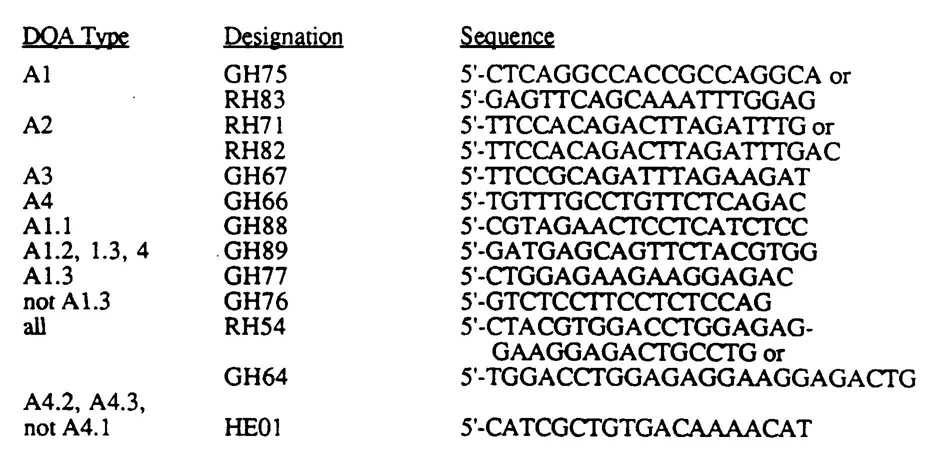

Although most of the probes are uniquely specific for one DQA type, two of the DQA1 subtyping probes cross hybridize to several DNA types. GH89 hybridizes to a sequence common to the DQA1.2, 1.3, and 4 types, and the probe GH76 detects all DQA types except DQA1.3. The GH76 probe is needed to distinguish DQA1.2/1.3 heterozygotes from DQA1.3/1.3 homozygotes. Further, the length and strand specificity of the probes were adjusted so that their relative hybridization efficiencies and stringency requirements for allelic discrimination were approximately the same. These eight probes produce a unique hybridization pattern for each of the 21 possible DQA diploid combinations.

The sequence variation that defines the DQalpha DNA types is localized within a relatively small hypervariable region of the second exon that can be encompassed within a single 242 bp PCR amplification product (see Horn et al., 1988, Proc. Natl. Acad. Sci. USA 85:6012-6016). These primers are shown below. 
Primer RS134 is 5'-GTGCTGCAGGTGTAAACTTGTACCAG
Primer RS135 is 5'-CACGGATCCGGTAGCAGCGGTAGAGTTG
 Biotinylated PCR primers were used to amplify this 242 bp DQalpha sequence from several genomic DNA samples: six homozygous cell lines and six heterozygous individuals.

The biotinylated primers were synthesized as follows. Primary amino groups were introduced at the 5' termini of the primers by a variation of the protocols set forth in Coull et al., 1986, Tetrahedron Lett. 27:3991-3994 and Connolly, 1987, Nuc. Acids Res. 15:3131-3139. Briefly, tetraethylene glycol was converted to the mono-phthalimido derivative by reaction with phthalimide in the presence of triphenylphosphine and diisopropyl azodicarboxylate (see Mitsunobu, 1981, Synthesis, pp. 1-28). The monophthalimide was converted to the corresponding beta-cyanoethyl diisopropylamino phosphoramidite as described in Sinha et al., 1984, Nuc. Acids Res. 12:4539. The resulting phthalimido amidite was added to the 5' ends of the oligonucleotides during the final cycle of automated DNA synthesis using standard coupling conditions. During normal deprotection of the DNA (concentrated aqueous ammonia for five hours at 55 degrees C), the phthalimido group was converted to a primary amine which was subsequently acylated with an appropriate biotin active ester. LC-NHS-biotin (Pierce) was selected for its water solubility and lack of steric hindrance. The biotinylation was performed on crude, deprotected oligonucleotide and the mixture purified by a combination of gel filtration and reversed-phase HPLC (see Levenson et al., 1989, in PCR Protocols and Applications - A Laboratory Manual, eds. Innis et al., Academic Press, NY).

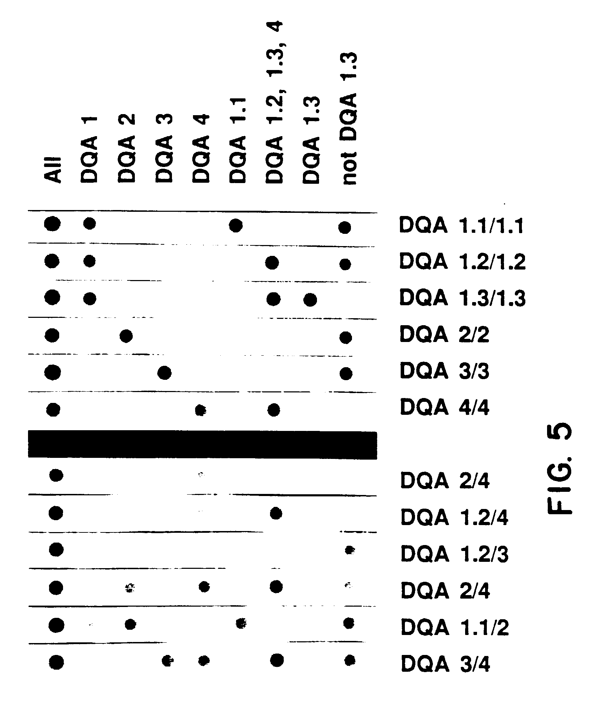

After hybridization of the amplified DNA to the membranes and color development, the DQalpha genotypes of these samples is readily apparent, as is shown in Figure 5. In Figure 5, the specificity of each immobilized probe is noted at the top of the filters and the DQA genotype of each sample is noted at the right of the corresponding filter.

The immobilized probes of the invention have so facilitated the method of DNA typing at the HLA DQalpha locus that kits for typing will be important commercially. These kits can come in a variety of forms, but a preferred embodiment of the kit is described in detail, below. This description is followed by a description of simplified typing protocols for use with the kit.

A preferred kit will contain one or more vials of pre-aliquoted, "sterilized" (see below) DQalpha PCR amplification mixes, typically in concentrated (2X is preferred) form and pre-aliquoted in 50 µl aliquots. Each 50 µl aliquot will contain: 5 µmol KCl, 1 µmol Tris-HCl (pH = 8.3), 250 nmol MgCl₂, 15 pmol of biotinylated RS134, 15 pmol of biotinylated RS134, 18.75 nmol each of dGTP, dATP, dTTP, dCTP, and from 2.5 units up to 50 units of recombinant Taq polymerase (PECI). The dNTPs will be prepared from stock solutions at pH = 7. The sterilization protocol also introduces very low levels of inactivated DNAse and NaCl, as noted below.

The "sterilized" reagents referred to above relate to the need to avoid contamination of reagents with non-sample-derived nucleic acid sequences. Because PCR is such a powerful amplification method, contaminating molecules can lead to error. To avoid this contamination problem, the present invention provides a novel sterilization procedure. This procedure employs a DNAse, preferably bovine pancreas DNAse, to remove low levels of DNA contamination from batches of PCR reaction mix. Because DNA primers are sensitive to this enzyme, the primers are omitted from the batch until the DNAse has been inactivated by thermal denaturation. However, if RNA primers are to be employed in the PCR mixture, the primers can be present during sterilization. In addition, derivatized nucleotides can be used to make an oligonucleotide resistant to DNAse; for instance, thio-substituted nucleotides, such as phosphorothioates can be used to prepare oligonucleotides resistant to DNAse (see Sitzer and Eckstein, 1988, Nuc. Acids Res. 16: 11,691). Those of skill in the art recognize that an equivalent sterilization procedure utilizes a restriction enzyme that cleaves a sequence present in the amplification target; if any contaminating target is present, the restriction enzyme will cleave the contaminant, rendering it unavailable for amplification.

In the preferred sterilization procedure, however, 2.5 ml of 10X Taq buffer (100 mM Tris-HCl, pH = 8.3; 500 mM KCI; and 25 mM MgCl₂) are autoclaved and added to 0.19 ml of a solution that is 25 mM in each dNTP, 0.13 ml of Taq DNA polymerase at a concentration of 5 U/µl, and 8.75 ml of glass distilled water. The mixing of these reagents can be conveniently carried out in a 50 ml polypropylene tube. Once the mixture is prepared, 650 U of DNAse I (Cooper Biomedicals; 2500 U/ml in 150 mM NaCl, stored frozen) are added and the resulting solution incubated at 37 degrees C for 15 minutes. The DNAse is inactivated by incubating the mixture at 93 degrees C for 10 minutes. Then, 0.38 ml of each primer at 10 µM is added to the sterilized reagent, which is then aliquoted, preferably with a "dedicated" pipettor and in the protected confines of a laminar flow hood.

The kit can optionally contain the PCR reagents above, but must contain the immobilized probes of the invention, which can be prepared as described above with a blotting and automated pipetting device. The solid support can be conveniently marked by silk-screening. The kit can also contain SA-HRP at a concentration of 20 µg/ml HRP, which correlates to 250 nmol/ml SA. The SA-HRP is supplied in a buffer composed of 10 mM ACES, 2 M NaCI, at a pH = 6.5. The kit can also contain a concentrated (5X to 20X) solution of chromogen, such as leuco dye (as is marketed by Kodak in the Sure-Cell^{TM} diagnostic kits) or TMB.

The kit will also be more successful if simple, easy-to-follow instructions are included. Typical instructions for a preferred embodiment of the present detection method are as follows. About 2 (if hybridization is carried out in a sealed bag) to 3 (if hybridization is carried out in a trough) ml of hybridization solution (5X SSPE, 0.5% SDS, and, in some instances, 1% dextran-sulfate (M.W. 500,000, although other M.W. forms would work) aids in color retention) are pre-warmed to 55 degrees C prior to use. The sample DNA is amplified by PCR using biotinylated primers, and the biotinylated product is heated to 95 degrees for 3 to 5 minutes to denature the DNA. Denaturation can also be accomplished by adding 5 µl of 5 M NaOH to 100 µl of PCR product (final NaOH concentration is 250 mM). About 15 µl of SA-HRP stock (20 µg/ml, stored at 4 degrees C and never frozen) are then added to the 2 to 3 ml of hybridization solution, and then, 20 µl of the still hot, denatured PCR product are added to the mixture. If alkali denaturation is used, then one needs to use more PCR product to maintin the same level of sensitivity attained with heat denaturation. Typically 25 to 50 µl of PCR product are used with 20 to 40 µl of the SA-HRP stock solution. Best results are obtained when the strepavidin and the biotin are in approximate molar equivalency, i.e., about 300 ng of SA-HRP (measured in HRP) are used for every 6 pmol of biotinylated PCR product used for hybridization. The PCR product should always be added last and immediately after denaturation.

If the hybridization is carried out in a sealed bag, all air bubbles should be removed prior to sealing the bag. If the hybridization is carried out in a trough, the entire trough should be firmly covered with a glass plate. Hybridization is carried out for 20 minutes at 55 degrees C in a shaking water bath set at a moderate to high shaking speed, i.e., 50 to 200 rpm. The wash solution (2X SSPE, 0.1% SDS) is pre-warmed to 55 degrees during the hybridization step. After hybridization, all filters are placed in a bowl containing 200 to 300 ml of pre-warmed wash solution and washed for 8 to 10 minutes in a shaking water bath at 55 degrees C.

Color development is accomplished at room temperature and usually in a shaking water bath as follows if the chromogen is TMB. The filters are rinsed in 200 to 300 ml of room temperature wash solution for 5 minutes, then transferred to 200 to 300 ml of Buffer C (100 mM NaCitrate, pH = 5.0) and rinsed for 5 minutes, then incubated for 5 minutes in 40 ml of Buffer C containing 2 ml of TMB (2 mg/ml in 100% ethanol and stored at 4 degrees C), then transferred to a fresh dye solution (composed of 40 ml of Buffer C and 2 ml of TMB) containing 4 µl of 30% hydrogen peroxide, and the color is allowed to develop for 5 to 15 minutes. The color development is stopped by rinsing the filters twice with water; the filters can be dried and stored if protected from light. If the typing is weak (faint dots), the procedure is repeated using 50 µl of the PCR product and 40 µl of the SA-HRP during the hybridization step. If leuco dye is used in place of TMB, then one replaces the Buffer C rinse with a rinse in 200 to 300 ml of lX PBS, after which the filters are placed in 25 ml of a mixture of the dye and hydrogen peroxide (the same formulation as in Kodak Sure-Cell^{TM} kits). The development time is 5 to 10 minutes; color development is stopped by washing the filters twice in PBS.

### Example 5

### Detection of Beta-thalassemia Mutations

Although there are over 54 characterized mutations of the beta-globin gene that can give rise to beta-thalassemia, each ethnic group in which this disease is prevalent has a limited number of common mutations (see Kazazian et al., 1984, Nature 310: 152-154; Kazazian et al, 1984, EMBO J. 3:593-596; and Zhang et al., 1988, Hum. Genet. 78:37-40). In Mediterranean populations, eight mutations are responsible for over 90% of the beta-thalassemia alleles.

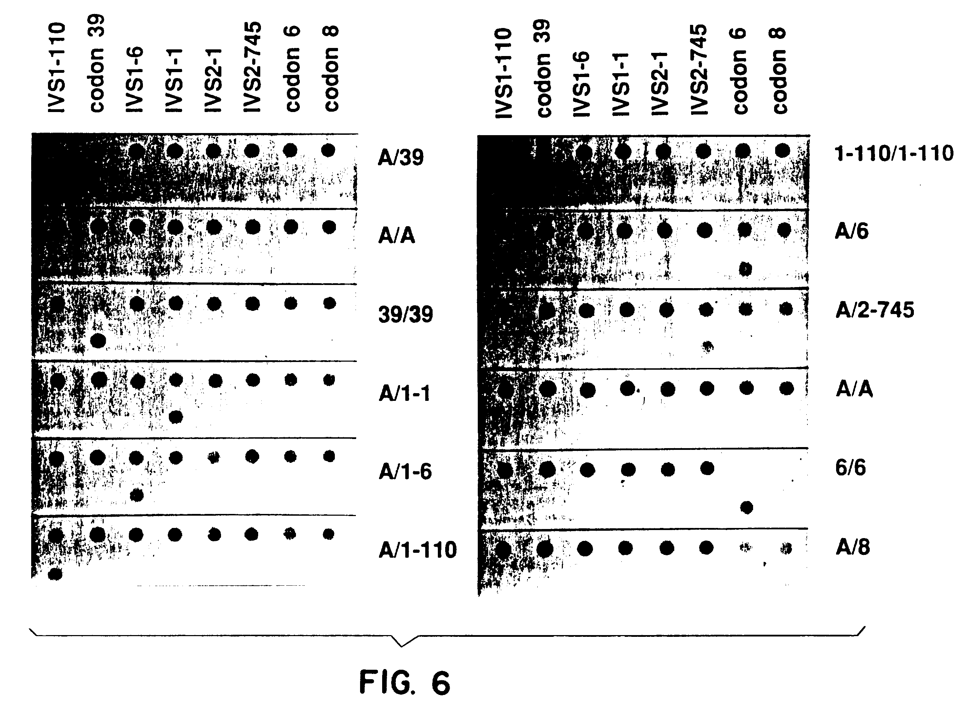

Probes were synthesized that are specific for each of these eight mutations as well as their corresponding normal sequences. The probes were given 400 nt poly-dT tails with terminal transferase and applied to membranes. Various amounts of each probe were applied to twelve duplicate nylon filters, irradiated at 40 mJ/cm², hybridized with amplified beta-globin sequences in genomic DNA samples, and color developed. The result is shown in Figure 6. In the figure, the beta-thalassemia locus that is detected by each immobilized probe pair is written at the top of the filters. For each filter, the upper row contains the probes that are specific for the normal sequence, and the lower row contains the probes specific for the mutant sequences. The beta-globin genotype of each sample is noted at the right of the corresponding filter. The name, amount applied to the membrane (in pmols and noted parenthetically), specificity, and sequence of each probe is shown below.

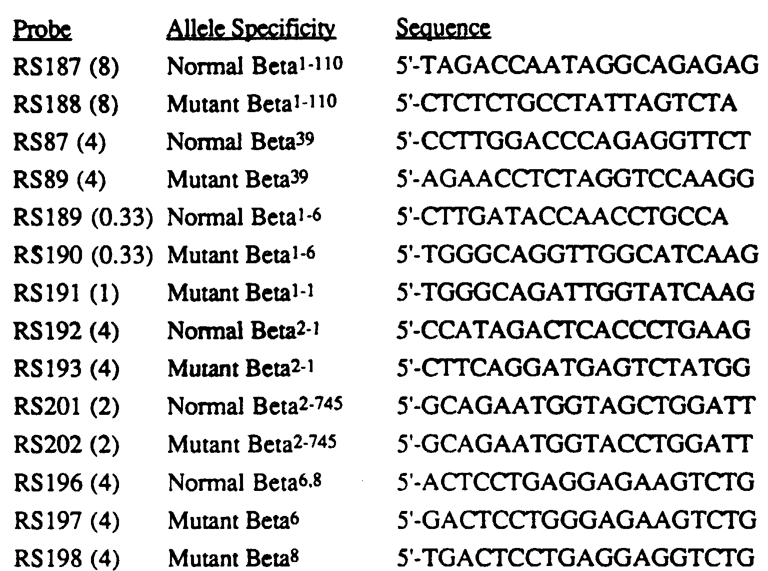

Because the beta-thalassemia mutations are distributed throughout the beta-globin gene, biotinylated PCR primers that amplify the entire gene in a single 1780 bp amplified product were used. The primers used for the amplification are shown below. 
RS151 is 5'-ATCACTTAGACCTCACCCTG
RS152 is 5'-GACCTCCCACATTCCCTTTT
 This amplification product encompasses all known beta-thalassemia mutations. Following hybridization and color development, the beta-globin genotypes could be determined by noting the pattern of hybridization, as shown in Figure 6.

Unlike the DQalpha typing system, two probes are needed to analyze each mutation -- one specific for the normal sequence and one specific for the mutant sequence -- to differentiate normal/mutant heterozygous carriers from mutant/mutant homozygotes. A complicating factor in this analysis is caused by apparent secondary structure in various portions of the relatively long beta-globin amplification product that interferes with probe hybridization. The relatively high stringency needed to minimize this secondary structure requires the use of longer (19 nt hybridizing regions) probes to capture the amplified beta-globin fragment. Because this constraint would not permit varying the length of the probes to compensate for different hybridization efficiencies, the balancing of signal intensities was accomplished by adjusting the amount of each oligonucleotide applied to the membrane.

## Claims

1. An assay reagent for diagnostic tests comprising at least one oligonucleotide probe, optionally an oligodeoxyribonucleotide probe, immobilized on a solid support having reactive groups, said probe comprising a hybridizing region composed of a nucleotide sequence of about 10 to 50 nucleotides complementary to a specific nucleotide sequence to be detected and a spacer arm characterized in that the immobilization of each hybridizing region is performed via the spacer arm which is longer than said nucleotide sequence and allows the hybridizing region to move away from the solid support and which spacer arm is not able to hybridize to the specific nucleotide sequence to be detected, wherein the spacer arm is covalently bonded at one side to said support and at the other side to the hybridization region.

2. An assay reagent of claim 1, wherein said spacer arm is a polynucleotide tail, optionally containing from 200 to 800 nucleotides and, if desired, comprising at least 150 pyrimidine nucleotides, said reactive groups are primary or secondary amine groups and said bonding between said tail and said support being formed by ultraviolet light irradiation.

3. An assay reagent of claim 1 or claim 2, characterized in that said support is a nylon support.

4. An assay reagent of any one of claims 1 to 3, characterized in that the hybridizing region is composed of a sequence of nucleotides from 17 to 23 nucleotides in length.

5. An assay reagent of any one of claims 1 to 4 charcterized in that it comprises a set of probes immobilized on the solid support, wherein said set of probes comprises two or more members, each member of said set having a hybridizing region different from every other member of said set and each member is immobilized on said solid support at a discrete location separate from every other member of said set and, if desired, one member of said set serving as a positive control.

6. An assay reagent according to claim 5 characterized in that the assay reagent also comprising a labeled polynucleotide hybridized to one of said probes, wherein said polynucleotide includes at least 50 nucleotides, or comprising a target sequence from a sample hybridized to a probe of said set and a colored or fluorescent compound immobilized on said support at the location of said hybridized probe.

7. An assay reagent of claim 5 or 6 characterized in that said probes of said set are complementary to nucleic acid sequences of microorganisms or are complementary to variant alleles of a genetic locus, optionally an HLA locus, especially DQualpha locus.

8. A method for preparing an assay reagent as defined by any one of the claims 1 to 7 characterized in that a nucleotide sequence of about 10 to 50 nucleotides complementary to a specific nucleotide sequence to be detected is attached by covalent bond to one end of a spacer arm which spacer arm is longer than said nucleotide sequence and is not able to hybridize to the specific nucleotide sequence to be detected, and the other end of the spacer arm is immobilized on a solid support having reactive groups by a covalent bond between the reactive groups and that end of said spacer arm and, if desired, said method comprising (a) contacting said solid support comprising amine groups as reactive groups and (b) irradiating the support prepared in step (a) with ultraviolet light, preferably having a wave-length of 254 nm.

9. A method for detecting the presence of a nucleic acid sequence in a sample, characterized in that the method comprises: (a) contacting said sample with an assay reagent of any one claims 1 to 7 under conditions that allow for hybridization of complementary nucleic acid sequences; and (b) determining if hybridization has occured.

10. A kit comprising an assay reagent of any one of claims 1 to 7.

## Patentansprüche

1. Testreagenz für diagnostische Tests, umfassend
mindestens eine Oligonucleotidsonde, gegebenenfalls eine Oligodesoxyribonucleotidsonde, die an einem festen Träger mit reaktiven Gruppen immobilisiert ist, wobei die Sonde eine hybridisierende Region umfaßt, zusammengesetzt aus einer Nucleotidsequenz von etwa 10 bis 50 Nucleotiden, die komplementär zu einer spezifischen nachzuweisenden Nucleotidsequenz sind, und einen Spacerarm, dadurch gekennzeichnet, daß die Immobilisierung jeder hybridisierenden Region durchgeführt wird mittels des Spacerarms, der länger ist als die Nucleotidsequenz und es der hybridisierenden Region ermöglicht, sich vom festen Träger wegzubewegen, und wobei der Spacerarm nicht fähig ist, an die nachzuweisende spezifische Nucleotidsequenz zu hybridisieren, wobei der Spacerarm an einer Seite an den Träger und an der anderen Seite an die Hybridisierungsregion gebunden ist.

2. Testreagenz nach Anspruch 1, wobei der Spacerarm ein Polynucleotidschwanz ist, der wahlweise zwischen 200 und 800 Nucleotiden enthält und, falls gewünscht, mindestens 150 Pyrimidinnucleotide umfaßt, wobei die reaktiven Gruppen primäre oder sekundäre Amingruppen sind und die Bindung zwischen dem Schwanz und dem Träger durch ultraviolette Lichtstrahlung erzeugt wird.

3. Testreagenz nach Anspruch 1 oder 2, dadurch gekennzeichnet, daß der Träger ein Nylonträger ist.

4. Testreagenz nach einem der Ansprüche 1 bis 3, dadurch gekennzeichnet, daß die hybridisierende Region aus einer Nudeotidsequenz von 17 bis 23 Nucleotiden Länge gebildet ist.

5. Testreagenz nach einem der Ansprüche 1 bis 4, dadurch gekennzeichnet, daß es umfaßt:
einen an dem festen Träger immobilisierten Sondensatz, wobei der Sondensatz zwei oder mehr Mitglieder umfaßt, jedes Mitglied des Satzes eine Hybridisierungsregion hat, die unterschiedlich zu jedem anderen Mitglied des Satzes ist, und jedes Mitglied an dem festen Träger an einer bestimmten Stelle, getrennt von jedem anderen Mitglied des Satzes immobilisiert ist und, falls gewünscht, ein Mitglied des Satzes als eine positive Kontrolle dient.

6. Testreagenz nach Anspruch 5, dadurch gekennzeichnet, daß das Testreagenz weiterhin ein markiertes Polynucleotid umfaßt, das mit einer der Sonden hybridisiert, wobei das Polynucleotid mindestens 50 Nucleotide beinhaltet, oder eine Zielsequenz von einer Probe umfaßt, die zu einer Sonde des Satzes hybridisiert, und eine farbige oder fluoreszierende Verbindung, immobilisiert an dem Träger am Ort der hybridisierten Sonde.

7. Testreagenz nach Anspruch 5 oder 6, dadurch gekennzeichnet, daß die Sonden des Satzes komplementär sind zu Nucleinsäuresequenzen von Mikroorganismen oder komplementär sind zu varianten Allelen eines genetischen Locus', wahlweise eines HLA-Locus, insbesondere des DQalpha-Locus.

8. Verfahren zur Herstellung eines Testreagenzes gemäß der Definition in einem der Ansprüche 1 bis 7, dadurch gekennzeichnet, daß eine Nucleotidsequenz von etwa 10 bis 50 Nucleotiden, komplementär zu einer spezifischen Nucleotidsequenz, die nachgewiesen werden soll, durch eine kovalente Bindung an einem Ende eines Spacerarms gebunden ist, wobei der Spacerarm länger ist als die Nucleotidsequenz und nicht fähig ist, an die nachzuweisende spezifische Nucleotidsequenz zu hybridisieren, und das andere Ende des Spacerarms an einem festen Träger, der reaktive Gruppen hat, durch eine kovalente Bindung zwischen den reaktiven Gruppen und dem Ende des besagten Spacerarmes immobilisiert ist und, falls gewünscht, das Verfahren umfaßt:
(a) Inkontaktbringen des festen Trägers, der Amingruppen als reaktive Gruppen umfaßt; und
(b) Bestrahlen des in Schritt (a) hergestellten Trägers mit ultraviolettem Licht, vorzugsweise mit einer Wellenlänge von 254 nm.

9. Verfahren zum Nachweis der Gegenwart einer Nucleinsäuresequenz in einer Probe, dadurch gekennzeichnet, daß das Verfahren umfaßt:
(a) Inkontaktbringen der Probe mit einem Testreagenz nach einem der Ansprüche 1 bis 7 unter Bedingungen, die die Hybridisierung der komplementären Nucleinsäuresequenzen erlauben; und
(b) Bestimmen, ob eine Hybridisierung stattgefunden hat.

10. Kit, der ein Testreagenz nach einem der Ansprüche 1 bis 7 umfaßt.

## Revendications

1. Réactif d'essai pour tests de diagnostic comprenant au moins une sonde oligonucléotidique, éventuellement une sonde oligodésoxyribonucléotidique, immobilisée sur un support solide comportant des groupes réactifs, ladite sonde comprenant une région d'hybridation composée d'une séquence nucléotidique d'environ 10 à 50 nucléotides, complémentaire d'une séquence nucléotidique déterminée à détecter, et un segment espaceur, caractérisé en ce que l'immobilisation de chaque région d'hybridation est effectuée par l'intermédiaire du segment espaceur qui est plus long que ladite séquence nucléotidique et permet à la région d'hybridation de s'écarter dudit support solide, et lequel segment espaceur n'est pas capable de s'hybrider avec la séquence nucléotidique déterminée à détecter, le segment espaceur étant lié par covalence d'un côté audit support et de l'autre côté à la région d'hybridation.

2. Réactif d'essai de la revendication 1, dans lequel ledit segment espaceur est une queue polynucléotidique, éventuellement contenant de 200 à 800 nucléotides et, si on le désire, comprenant au moins 150 nucléotides à pyrimidines, lesdits groupes réactifs sont des groupes amino primaires ou secondaires et ladite liaison entre ladite queue et ledit support étant formée par irradiation à la lumière ultraviolette.

3. Réactif d'essai de la revendication 1 ou 2, caractérisé en ce que ledit support est un support en Nylon.

4. Réactif d'essai de l'une quelconque des revendications 1 à 3, caractérisé en ce que la région d'hybridation est composée d'une séquence de nucléotides de 17 à 23 nucléotides de longueur.

5. Réactif d'essai de l'une quelconque des revendications 1 à 4, caractérisé en ce qu'il comprend une série de sondes immobilisées sur le support solide, ladite série de sondes comprenant deux membres ou plus, chaque membre de ladite série ayant une région d'hybridation différente de tout autre membre de ladite série et chaque membre étant immobilisé sur ledit support solide en un emplacement distinct, séparé de tout autre membre de ladite série et, si on le désire, un membre de ladite série servant de témoin positif.

6. Réactif d'essai selon la revendication 5, caractérisé en ce que le réactif d'essai comprend également un polynucléotide marqué hybridé avec l'une desdites sondes, ledit polynucléotide comprenant au moins 50 nucléotides, ou comprend une séquence cible provenant d'un échantillon hybridé avec une sonde de ladite série, et un composé coloré ou fluorescent immobilisé sur ledit support à l'emplacement de ladite sonde hybridé.

7. Réactif d'essai de la revendication 5 ou 6, caractérisé en ce que lesdites sondes de ladite série sont complémentaires de séquences d'acide nucléique de micro-organismes ou sont complémentaires d'allèles variants d'un locus génétique, éventuellement un locus HLA, en particulier le locus DQualpha.

8. Procédé de préparation d'un réactif d'essai tel que défini par l'une quelconque des revendications 1 à 7, caractérisé en ce qu'une séquence nucléotidique d'environ 10 à 50 nucléotides, complémentaire d'une séquence nucléotidique déterminée à détecter, est attachée par liaison covalente à une extrémité d'un segment espaceur, lequel segment espaceur est plus long que ladite séquence nucléotidique et n'est pas capable de s'hybrider avec la séquence nucléotidique déterminée à détecter, et l'autre extrémité du segment espaceur est immobilisée sur un support solide comportant des groupes réactifs, par une liaison covalente entre les groupes réactifs et cette extrémité dudit segment espaceur et, si on le désire, ledit procédé comprenant (a) la mise en contact dudit support solide comprenant des groupes amino en tant que groupes réactifs et (b) l'irradiation du support préparé dans l'étape (a) avec de la lumière ultraviolette, ayant de préférence une longueur d'onde de 254 nm.

9. Procédé de détection de la présence d'une séquence d'acide nucléique dans un échantillon, caractérisé en ce que le procédé comprend: (a) la mise en contact dudit échantillon avec un réactif d'essai de l'une quelconque des revendications 1 à 7, dans des conditions permettant l'hybridation de séquences d'acide nucléique complémentaires; et (b) le fait de déterminer si une hybridation s'est produite.

10. Nécessaire comprenant un réactif d'essai de l'une quelconque des revendications 1 à 7.
